# EUROPEAN PATENT APPLICATION

(11) **EP 4 545 631 A1**
(43) Date of publication of application: **30.04.2025**
(21) Application number: 23827571.3
(22) Date of filing: 23.06.2023
(51) Int. Cl.: C12N 5/078, C07K 14/725, C07K 16/28, A61K 35/17, A61P 35/00

(54) **IMMUNE CELL EXPRESSING IMMUNE CHECKPOINT REGULATOR, AND USE THEREOF**

(30) Priority: 24.06.2022 KR 20220077551
(71) Applicant: HK inno.N Corporation, Cheongju-si, Chungcheongbuk-do 28158 (KR)
(72) Inventor: KWON, Ki Mun, Seoul 04551 (KR); LEE, Bo Eun, Seoul 04551 (KR); KIM, Ji Eun, Seoul 04551 (KR); YANG, Yoo Hee, Seoul 04551 (KR); KIM, Chak Hee, Seoul 04551 (KR); BYUN, Sung Hyun, Seoul 04551 (KR)
(74) Representative: Maiwald GmbH
(86) International application number: PCT/KR2023/008788
(87) International publication number: WO 2023/249463

(57) **Abstract**

The present invention relates to an immune cell expressing an immune checkpoint regulator so as to overcome an immune checkpoint, a preparation method therefor, and a pharmaceutical composition comprising the immune cell, and, more specifically, to an immune-checkpoint-overcoming immune cell, a preparation method therefor, and a pharmaceutical composition comprising the immune cell, the immune-checkpoint-overcoming immune cell being genetically modified so that an anti-HLA-G soluble scFv specifically binding to HLA-G, which is an immune checkpoint, is expressed together with an antigen recognition receptor. If an anti-HLA-G soluble scFv of the present invention is expressed alone or simultaneously with an antigen recognition receptor in T lymphocytes, natural killer cells, and the like, immune cells inhibited by cancer cells expressing an immune checkpoint are activated so as to more effectively remove cancer cells such that even the activation of endogenous immune cells, which had been inhibited by immune checkpoints, can be expected, and thus the present invention can be effectively used as an immunotherapeutic method for various cancer diseases associated with HLA-G.

## Description

### [Technical Field]

The present invention relates to an immune cell expressing a factor capable of controlling an immune checkpoint, an expression vector for preparing the same, a composition including the same, and a use thereof.

### [Background Art]

Methods for treating cancer have undergone a process of constant development and change, and methods such as surgery, chemotherapy, radiotherapy, and the like are still used to this day. However, these existing cancer treatment methods are mostly effective only in the early stages when cancer has not metastasized, and there is a problem that in cases where metastasis has already occurred, there is a high possibility of recurrence even after surgery is performed. Therefore, there is ongoing research on methods for using immune responses to treat cancer.

In recent years, the therapeutic potential of T cells expressing a chimeric antigen receptor (CAR-T) in the treatment of blood cancers has been proven, and CAR-T therapies targeting CD 19 or a B-cell maturation antigen (BCMA) have been commercially available. However, due to the characteristics of solid tumors that are different from blood cancers, especially the tumor microenvironment (TME) that suppresses immune responses, additional strategies capable of effectively targeting the TME to treat cancer are needed in addition to solid tumor CAR-T therapies.

Meanwhile, human leukocyte antigen G (HLA-G) is a non-classical HLA class I molecule that is known to be specifically expressed only in the extravillous cytotrophoblast of the placental bed. Also, it is thought that this expression plays an important role in maintaining immune tolerance between the fetus and the mother.

Known immune cell receptors for HLA-G include Ig-like transcript 2 (ILT2), ILT4, and killer cell immunoglobulin-like receptor, two Ig domains and long cytoplasmic tail 4 (KIR2DL4), and all of these receptors have an immunoreceptor tyrosine-based inhibitory motif (ITIM motif). When HLA-G binds to the receptor, this motif activates a signaling system that suppresses immune cells.

HLA-G is expressed in a limited manner in normal cells, but overexpressed in several types of cancer (Lin A, Yan WH. Human Leukocyte Antigen-G (HLA-G) Expression in Cancers: Roles in Immune Evasion, Metastasis and Target for Therapy. Mol. Med. 21(1), 782-791 (2015)), which is associated with immune suppressive microenvironments, advanced cancer stage, insufficient treatment response, or prognosis.

Also, HLA-G may inhibit the efficacy of endogenous immune cells as well as immunotherapeutic agents targeting specific antigens due to its characteristic of being overexpressed in various types of cancer. In other words, HLA-G may reduce the efficacy of endogenous and exogenous immune cells because it acts as an immune checkpoint that suppresses immunity. Therefore, there is a need for a method that can overcome the immune checkpoint function of HLA-G so as to effectively treat cancer using immune responses.

### [Disclosure]

### [Technical Problem]

In the above circumstances, the present inventors of the present invention have made extensive research efforts to develop immune cells capable of overcoming the tumor microenvironment of human leukocyte antigen G (HLA-G)-overexpressing cancers, especially solid cancers. As a result, the present inventors have manufactured immune cells that express and secrete soluble scFv derived from anti-HLA-G antibodies that specifically bind to HLA-G and found that the manufactured immune cells exhibit higher anti-cancer activity than general immune cells against cell lines expressing HLA-G, and thus have completed the present invention.

According to the present invention, the immune cell of the present invention can improve the microenvironment of tumor to activate immune cells such as tumor-infiltrating lymphocytes and may also further induce the activity of endogenous immune cells because it secretes a soluble single-chain Fv (scFv) capable of inhibiting the interaction between HLA-G and a receptor thereof. Therefore, the immune cell of the present invention can more efficiently remove cancer cells, and can be used for the treatment of various cancers as well as cancers associated with high expression of HLA-G.

Accordingly, the present invention is directed to providing an immune cell having high anti-tumor activity by expressing a soluble scFv that specifically binds to a target selected from the group consisting of HLA-G and a receptor thereof, which have immunosuppressive activity.

Also, the present invention is directed to providing an immune cell that further expresses an antigen recognition receptor.

In addition, the present invention is directed to providing an expression vector for preparing the immune cell.

Further, the present invention is directed to providing a pharmaceutical composition for treating cancer, which includes the immune cell and a pharmaceutically acceptable carrier.

### [Technical Solution]

To solve the above problems, according to an aspect of the present invention, there is provided an immune cell expressing and secreting:
an antigen recognition receptor; and
an antibody or an antigen binding fragment thereof that specifically binds to a target selected from the group consisting of a human leukocyte antigen G (HLA-G) and a receptor thereof.

According to another aspect of the present invention, there is provided an immune cell expressing and secreting an antibody or an antigen binding fragment thereof that specifically binds to a target selected from the group consisting of human leukocyte antigen G (HLA-G) and receptor thereof.

In the present invention, the immune cell may be selected from the group consisting of natural killer cells, T cells, B cells, macrophages, dendritic cells, natural killer dendritic cells, mast cells, and precursor cells thereof. More specifically, the immune cell may be an effector cell such as a T cell or a B cell.

In the present invention, the immune cell includes a population of autologous cells or a population of allogeneic cells. That is, the immune cell includes a population of autologous cells or a population of allogeneic cells that express and secrete an antibody or an antigen binding fragment thereof that specifically binds to a target selected from the group consisting of human leukocyte antigen G (HLA-G) and a receptor thereof.

In the present specification, the term "autologous" refers to any material derived from the same subject to which the material is to be reintroduced. In the present specification, the term "allogeneic" refers to any material derived from a different animal of the same species as the subject to which the material is to be introduced.

In the present invention, the difference between the two immune cells is the presence or absence of an antigen recognition receptor. In the present invention, the antigen recognition receptor is a receptor that literally recognizes and binds to a specific antigen present in a cell. In this case, the antigen recognition receptor activates the immune cell when it is bound to the antigen.

In the present invention, the antigen recognition receptor may be exogenous or endogenous.

The exogenous antigen recognition receptor refers to a receptor that is not naturally expressed in the relevant immune cells, but expressed by artificially manufacturing a receptor construct, such as a chimeric antigen receptor (CAR), and then delivering the receptor construct to the immune cells.

The endogenous antigen recognition receptor refers to a receptor naturally expressed in the relevant immune cells, and examples include T cell receptors (TCRs) or activating receptors such as NKG2D, which recognizes abnormal cells possessed by NK cells.

The distinction between the exogenous and endogenous antigen receptors is relative: In T cells, the TCR corresponds to an endogenous antigen receptor, whereas the TCR expressed in other immune cells corresponds to an exogenous antigen receptor.

The antigen recognition receptor may generally be composed of an antigen binding domain, a signal sequence (a leader sequence (LS)), a hinge, a transmembrane domain (TM), and an intracellular signaling domain (ICD), but the structure or configuration thereof is not limited to the above-mentioned range.

The antigen binding domain recognizes an antigen of a target cell and may be an antibody or an antibody fragment. The signal sequence refers to a sequence that allows the antigen recognition receptor to move to the cell membrane so that it can be expressed and exposed on the cell surface, and the transmembrane domain refers to a domain that connects the antigen binding domain and the intracellular signaling domain across the cell membrane.

In the present invention, the signal sequence, the hinge, the transmembrane domain, and the intracellular transport domain used in the antigen recognition receptor may be those commonly used in the art to which the present invention pertains.

In the present invention, the antigen recognition receptor may bind to only one antigen or ligand, or may bind to two or more antigens or ligands. The antigen binding domain may be selected from an antibody that recognizes a target antigen or a molecule that interacts with the antigen. Such an antigen includes, for example, a viral antigen, a bacterial (especially infectious bacterial) antigen, a parasitic antigen, a cell surface marker (for example, a tumor antigen) on a target cell associated with a specific pathology, or a surface molecule of an immune-related cell.

Specifically, the antigen that the antigen recognition receptor may recognize may be selected from the group consisting of CD19, CD20, CD22, CD7, CD10, CD30, CD33, CD34, CD38, CD41, CD44, CD49f, CD56, CD74, CD123, CD133, CD138, CD171, an epidermal growth factor receptor (EGFR), a prostate-specific membrane antigen (PSMA), GD2, an EGFR variant, tyrosine-protein kinase transmembrane receptor 1 (ROR1), c-Met, human epidermal growth factor receptor-2 (HER2), a carcinoembryonic antigen (CEA), mesothelin, GM2, MUC16, MUC1, CS1(CD319), interleukin 13 receptor alpha 2 (IL-13R a2), a B-cell maturation antigen (BCMA), Lewis Y, an IgG kappa chain, folate receptor-alpha, a prostate stem cell antigen (PSCA), an epithelial cell adhesion molecule (EpCAM), New York esophageal squamous cell carcinoma 1 (NY-ES0-1), Wilms' tumor gene 1 (WT-1), MAGE family member A1 (MAGE-A1), tumor associated glycoprotein-72 (TAG-72), killer cell lectin like receptor K1 (NKG2D(KLRK1), Claudin 18.2, Claudin 3, Claudin 4, and Claudin 6, but the present invention is not limited thereto.

The two immune cells are characterized by expressing and secreting an antibody or an antigen binding fragment thereof that specifically binds to a target selected from the group consisting of human leukocyte antigen G (HLA-G) and a receptor thereof.

HLA-G is overexpressed in various types of cancer, and known receptors for HLA-G in immune cells include Ig-like transcript 2 (ILT2), ILT4, and killer cell immunoglobulin like receptor, two Ig domains and long cytoplasmic tail 4 (KIR2DL4), and the like. Since all of the receptors have an immunoreceptor tyrosine-based inhibitory motif (ITIM motif), a signaling system that suppresses immune cells is activated due to this motif when HLA-G binds to the receptors.

The antibody or antigen binding fragment thereof that specifically binds to the human leukocyte antigen G (HLA-G) or receptor thereof acts to block the binding of HLA-G to the receptors, and thus prevents the activation of a signaling system that suppresses immune cells, thereby enabling immune cells to overcome the immune checkpoint.

According to one exemplary embodiment of the present invention, the antibody or antigen binding fragment thereof that specifically binds to the human leukocyte antigen G (HLA-G) may include a sequence selected from the following:
(a) a heavy-chain variable region including heavy-chain complementarity determining region 1 (CDR1) having an amino acid sequence of SEQ ID NO: 21, heavy-chain CDR2 having an amino acid sequence of SEQ ID NO: 22, and heavy-chain CDR3 having an amino acid sequence of SEQ ID NO: 23; and
   a light-chain variable region including light-chain CDR1 having an amino acid sequence of SEQ ID NO: 24, light-chain CDR2 having an amino acid sequence of SEQ ID NO: 25, and light-chain CDR3 having an amino acid sequence of SEQ ID NO: 26;
(b) a heavy-chain variable region including heavy-chain CDR1 having an amino acid sequence of SEQ ID NO: 27, heavy-chain CDR2 having an amino acid sequence of SEQ ID NO: 28, and heavy-chain CDR3 having an amino acid sequence of SEQ ID NO: 29; and
   a light-chain variable region including light-chain CDR1 having an amino acid sequence of SEQ ID NO: 30, light-chain CDR2 having an amino acid sequence of SEQ ID NO: 31, and light-chain CDR3 having an amino acid sequence of SEQ ID NO: 32; or
(c) a heavy-chain variable region including heavy-chain CDR1 having an amino acid sequence of SEQ ID NO: 33, heavy-chain CDR2 having an amino acid sequence of SEQ ID NO: 34, and heavy-chain CDR3 having an amino acid sequence of SEQ ID NO: 35; and
   a light-chain variable region including light-chain CDR1 having an amino acid sequence of SEQ ID NO: 36, light-chain CDR2 having an amino acid sequence of SEQ ID NO: 37, and light-chain CDR3 having an amino acid sequence of SEQ ID NO: 38.

More specifically, the antibody or antigen binding fragment thereof that specifically binds to the human leukocyte antigen G (HLA-G) may include a sequence selected from the following:
(a) a heavy-chain variable region having an amino acid sequence of SEQ ID NO: 15 and a light-chain variable region having an amino acid sequence of SEQ ID NO: 16;
(b) a heavy-chain variable region having an amino acid sequence of SEQ ID NO: 17 and a light-chain variable region having an amino acid sequence of SEQ ID NO: 18; or
(c) a heavy-chain variable region having an amino acid sequence of SEQ ID NO: 19 and a light-chain variable region having an amino acid sequence of SEQ ID NO: 20.

In the present invention, the antibody or antigen binding fragment thereof that specifically binds to the human leukocyte antigen G (HLA-G) may include a sequence selected from the following:
(a) a heavy-chain variable region having an amino acid sequence of SEQ ID NO: 15 or a sequence identity of at least 80% or more, preferably 90% or more, more preferably 95% or more, and most preferably 99% or more with the amino acid sequence; and
   a light-chain variable region having an amino acid sequence of SEQ ID NO: 16 or a sequence identity of at least 80% or more, preferably 90% or more, more preferably 95% or more, and most preferably 99% or more with the amino acid sequence;
(b) a heavy-chain variable region having an amino acid sequence of SEQ ID NO: 17 or a sequence identity of at least 80% or more, preferably 90% or more, more preferably 95% or more, and most preferably 99% or more with the amino acid sequence; and
   a light-chain variable region having an amino acid sequence of SEQ ID NO: 18 or a sequence identity of at least 80% or more, preferably 90% or more, more preferably 95% or more, and most preferably 99% or more with the amino acid sequence; or
(c) a heavy-chain variable region having an amino acid sequence of SEQ ID NO: 19 or a sequence identity of at least 80% or more, preferably 90% or more, more preferably 95% or more, and most preferably 99% or more with the amino acid sequence; and
   a light-chain variable region having an amino acid sequence of SEQ ID NO: 20 or a sequence identity of at least 80% or more, preferably 90% or more, more preferably 95% or more, and most preferably 99% or more with the amino acid sequence.

More specifically, the antibody or antigen binding fragment thereof that specifically binds to the human leukocyte antigen G (HLA-G) may include an amino acid sequence selected from the group consisting of SEQ ID NOS: 1 to 3, and may include an amino acid sequence selected from the group consisting of SEQ ID NOS: 4 to 6.

Also, in the present invention, the antibody or antigen binding fragment thereof that specifically binds to the human leukocyte antigen G (HLA-G) may include an amino acid sequence selected from the group consisting of SEQ ID NOS: 1 to 3, or an amino acid sequence having a sequence identity of at least 80% or more, preferably 90% or more, more preferably 95% or more, and most preferably 99% or more with the amino acid sequence, and may also include an amino acid sequence selected from the group consisting of SEQ ID NOS: 4 to 6, or an amino acid sequence having a sequence identity of at least 80% or more, preferably 90% or more, more preferably 95% or more, and most preferably 99% or more with the amino acid sequence.

In the present invention, the term "antibody" refers to a protein molecule that acts as a receptor that specifically recognizes a specific antigen, including an immunoglobulin molecule that immunologically has reactivity with the antigen. The antibody includes a monoclonal antibody, a polyclonal antibody, a mixture of monoclonal and/or polyclonal antibodies, a full-length antibody, and an antibody fragment. A full-length antibody or an intact antibody is used interchangeably to refer to an antibody having a structure substantially similar to a native antibody structure, or having a heavy chain containing an Fc region as defined herein.

Also, the antibody may include bivalent or bispecific molecules (e.g., bispecific antibodies). In addition, the antibody may be a human antibody, a humanized antibody, or a chimeric antibody, depending on the origin of its sequence.

In the present invention, the term "monoclonal antibody" refers to an antibody molecule having a single molecular composition obtained from a population of substantially identical antibodies, and such a monoclonal antibody exhibits single binding avidity and affinity for a specific epitope, unlike polyclonal antibodies which may bind to multiple epitopes. In the present invention, the term "full-length antibody" refers to a structure having two full-length light chains and two full-length heavy chains, each light chain being linked to the heavy chain by a disulfide bond. The heavy chain constant region has gamma (γ), mu (µ), alpha (α), delta (δ), and epsilon (ε) types and has gamma 1 (γ1), gamma 2 (γ2), gamma 3 (γ3), gamma 4 (γ4), alpha 1 (α1), and alpha 2 (α2) subclasses. The constant region of the light chain has kappa (κ) and lambda (λ) types. IgG has subtypes, including IgG1, IgG2, IgG3, and IgG4.

In the present invention, the term "heavy chain" refers to both a full-length heavy chain and fragments thereof, which include a variable region domain VH including an amino acid sequence of a variable region sufficient to confer specificity to an antigen, and three constant region domains CH1, CH2 and CH3.

In the present invention, the term "light chain" refers to both a full-length light chain and fragments thereof, which include a variable region domain VL including an amino acid sequence of a variable region sufficient to confer specificity to an antigen, and a constant region domain CL.

In the present invention, the term "fragment," "antibody fragment," "antigen binding fragment," or "antigen binding domain" is used interchangeably to refer to any fragment of an antibody that retains the antigen binding function of the antibody. Exemplary antigen binding fragments include Fab, Fab', scFV, F(ab')₂, and Fv, but the present invention is not limited thereto.

The Fab has one antigen binding site with a structure having variable regions of the light and heavy chains, a constant region of the light chain, and the first constant region (CH1 domain) of the heavy chain. The Fab' differs from the Fab in that it has a hinge region containing one or more cysteine residues at the C-terminus of the heavy chain CH1 domain. The F(ab')₂ antibody is produced when cysteine residues in the hinge region of Fab' form a disulfide bond. The Fv is the minimum antibody fragment that has only a heavy-chain variable region and a light-chain variable region.

In the present invention, the antibody or antigen binding fragment thereof that specifically binds to the human leukocyte antigen G (HLA-G) may include not only the sequence of the anti-HLA-G scFv described in the present specification, but also biological equivalents thereof, within the range of being able to exhibit binding ability to HLA-G. For example, additional changes may be made to the amino acid sequence of the antibody in order to further improve the binding affinity and/or other biological properties of the antibody. Such modifications include, for example, deletions, insertions, and/or substitutions of amino acid sequence residues of the antibody. Such amino acid mutations are made based on the relative similarity of the amino acid side chain substituents, such as hydrophobicity, hydrophilicity, charge, size, and the like. Analysis of the size, shape, and type of the amino acid side chain substituents reveals that arginine, lysine, and histidine are all positively charged residues; alanine, glycine, and serine have similar sizes; and phenylalanine, tryptophan, and tyrosine have similar shapes. Therefore, based on these advantages, arginine, lysine and histidine; alanine, glycine and serine; and phenylalanine, tryptophan and tyrosine may be said to be biologically functional equivalents, respectively.

According to one exemplary embodiment of the present invention, the antibody or antigen binding fragment thereof that specifically binds to the human leukocyte antigen G (HLA-G) may be a single-chain variable fragment (Fv) (scFv) including the heavy-chain variable and light-chain variable sequences selected above, and specifically, may be a soluble single-chain Fv.

The single-chain Fv is a type of antibody fragment in which the variable regions of the antibody heavy and light chains are connected by a short linker of 10 to 25 amino acids.

According to one exemplary embodiment of the present invention, the scFv that specifically binds to the human leukocyte antigen G (HLA-G) may be expressed and secreted from an immune cell by a nucleic acid consisting of a nucleotide sequence encoding the scFv introduced from outside the cell.

Accordingly, the immune cell may include a nucleic acid having a nucleotide sequence encoding a scFv that specifically binds to the human leukocyte antigen G (HLA-G), wherein the nucleotide sequence may be selected from the following:
a heavy-chain variable region-coding nucleotide sequence including a nucleotide sequence encoding heavy-chain CDR1 including a nucleotide sequence encoding an amino acid sequence of SEQ ID NO: 21, 27, or 33; a nucleotide sequence encoding heavy-chain CDR2 including a nucleotide sequence encoding an amino acid sequence of SEQ ID NO: 22, 28, or 34; and a nucleotide sequence encoding heavy-chain CDR3 including a nucleotide sequence encoding an amino acid sequence of SEQ ID NO: 23, 29, or 35; and
a light-chain variable region-coding nucleotide sequence including a nucleotide sequence encoding light-chain CDR1 including a nucleotide sequence encoding an amino acid sequence of SEQ ID NO: 24, 30, or 36; a nucleotide sequence encoding light-chain CDR2 including a nucleotide sequence encoding an amino acid sequence of SEQ ID NO: 25, 31, or 37; and a nucleotide sequence encoding light-chain CDR3 including a nucleotide sequence encoding an amino acid sequence of SEQ ID NO: 26, 32, or 38.

More specifically, the nucleotide sequence encoding the scFv that specifically binds to the human leukocyte antigen G (HLA-G) may be selected from the following:
(a) a nucleotide sequence encoding heavy-chain CDR1 including a nucleotide sequence encoding the amino acid sequence of SEQ ID NO: 21, a nucleotide sequence encoding heavy-chain CDR2 including a nucleotide sequence encoding the amino acid sequence of SEQ ID NO: 22, and a nucleotide sequence encoding heavy-chain CDR3 including a nucleotide sequence encoding the amino acid sequence of SEQ ID NO: 23; and
   a nucleotide sequence encoding light-chain CDR1 including a nucleotide sequence encoding the amino acid sequence of SEQ ID NO: 24, a nucleotide sequence encoding light-chain CDR2 including a nucleotide sequence encoding the amino acid sequence of SEQ ID NO: 25, and a nucleotide sequence encoding light-chain CDR3 including a nucleotide sequence encoding the amino acid sequence of SEQ ID NO: 26;
(b) a nucleotide sequence encoding heavy-chain CDR1 including a nucleotide sequence encoding the amino acid sequence of SEQ ID NO: 27, a nucleotide sequence encoding heavy-chain CDR2 including a nucleotide sequence encoding the amino acid sequence of SEQ ID NO: 28, and a nucleotide sequence encoding heavy-chain CDR3 including a nucleotide sequence encoding the amino acid sequence of SEQ ID NO: 29; and
   a nucleotide sequence encoding light-chain CDR1 including a nucleotide sequence encoding the amino acid sequence of SEQ ID NO: 30, a nucleotide sequence encoding light-chain CDR2 including a nucleotide sequence encoding the amino acid sequence of SEQ ID NO: 31, and a nucleotide sequence encoding light-chain CDR3 including a nucleotide sequence encoding the amino acid sequence of SEQ ID NO: 32; or
(c) a nucleotide sequence encoding heavy-chain CDR1 including a nucleotide sequence encoding the amino acid sequence of SEQ ID NO: 33, a nucleotide sequence encoding heavy-chain CDR2 including a nucleotide sequence encoding the amino acid sequence of SEQ ID NO: 34, and a nucleotide sequence encoding heavy-chain CDR3 including a nucleotide sequence encoding the amino acid sequence of SEQ ID NO: 35; and
   a nucleotide sequence encoding light-chain CDR1 including a nucleotide sequence encoding the amino acid sequence of SEQ ID NO: 36, a nucleotide sequence encoding light-chain CDR2 including a nucleotide sequence encoding the amino acid sequence of SEQ ID NO: 37, and a nucleotide sequence encoding light-chain CDR3 including a nucleotide sequence encoding the amino acid sequence of SEQ ID NO: 38.

More specifically, the nucleotide sequence encoding the soluble scFv that specifically binds to the human leukocyte antigen G (HLA-G) may be selected from the following:
(a) a nucleotide sequence encoding a heavy-chain variable region having an amino acid sequence of SEQ ID NO: 15 and a nucleotide sequence encoding a light-chain variable region having an amino acid sequence of SEQ ID NO: 16;
(b) a nucleotide sequence encoding a heavy-chain variable region having an amino acid sequence of SEQ ID NO: 17 and a nucleotide sequence encoding a light-chain variable region having an amino acid sequence of SEQ ID NO: 18; or
(c) a nucleotide sequence encoding a heavy-chain variable region having an amino acid sequence of SEQ ID NO: 19 and a nucleotide sequence encoding a light-chain variable region having an amino acid sequence of SEQ ID NO: 20.

In the present invention, the nucleotide sequence encoding the soluble scFv may include:
a nucleotide sequence encoding a heavy-chain variable region including a nucleotide sequence encoding the amino acid sequence of SEQ ID NO: 15, 17, or 19 or a variant thereof having a sequence identity of at least 80% or more, preferably 90% or more, more preferably 95% or more, and most preferably 99% or more with the amino acid sequence; and
a nucleotide sequence encoding a light-chain variable region including a nucleotide sequence encoding the amino acid sequence of SEQ ID NO: 16, 18, or 20 or a variant thereof having a sequence identity of at least 80% or more, preferably 90% or more, more preferably 95% or more, and most preferably 99% or more with the amino acid sequence.

In the present invention, the nucleotide sequence encoding the soluble scFv may be selected from the following:
(a) a nucleotide sequence encoding a heavy-chain variable region including the amino acid sequence of SEQ ID NO: 15, or an amino acid sequence having a sequence identity of 80% or more, preferably 90% or more, more preferably 95% or more, and most preferably 99% or more with SEQ ID NO: 15, and a nucleotide sequence encoding a light-chain variable region including the amino acid sequence of SEQ ID NO: 16, or an amino acid sequence having a sequence identity of 80% or more, preferably 90% or more, more preferably 95% or more, and most preferably 99% or more with SEQ ID NO: 16;
(b) a nucleotide sequence encoding a heavy-chain variable region including the amino acid sequence of SEQ ID NO: 17, or an amino acid sequence having a sequence identity of 80% or more, preferably 90% or more, more preferably 95% or more, and most preferably 99% or more with SEQ ID NO: 17, and a nucleotide sequence encoding a light-chain variable region including the amino acid sequence of SEQ ID NO: 18, or an amino acid sequence having a sequence identity of 80% or more, preferably 90% or more, more preferably 95% or more, and most preferably 99% or more with SEQ ID NO: 18; or
(c) a nucleotide sequence encoding a heavy-chain variable region including the amino acid sequence of SEQ ID NO: 19, or an amino acid sequence having a sequence identity of 80% or more, preferably 90% or more, more preferably 95% or more, and most preferably 99% or more with SEQ ID NO: 19, and a nucleotide sequence encoding a light-chain variable region including the amino acid sequence of SEQ ID NO: 20, or an amino acid sequence having a sequence identity of 80% or more, preferably 90% or more, more preferably 95% or more, and most preferably 99% or more with SEQ ID NO: 20.

Meanwhile, the present inventors constructed an expression vector to express soluble scFv that specifically binds to the human leukocyte antigen G (HLA-G) in immune cells or to express the soluble scFv and an antigen recognition receptor.

Accordingly, another aspect of the present invention provides the following expression vector for preparing the immune cell:
(a) an expression vector including a nucleotide sequence encoding an antigen recognition receptor; and a nucleotide sequence encoding an antibody or an antigen binding fragment thereof that specifically binds to a target selected from the group consisting of human leukocyte antigen G and a receptor thereof;
(b) an expression vector including a nucleotide sequence encoding an antibody or an antigen binding fragment thereof that specifically binds to a target selected from the group consisting of human leukocyte antigen G and a receptor thereof; and
(c) two expression vectors of (c-1) and (c-2) below:
   (c-1) an expression vector containing a nucleotide sequence encoding an antigen recognition receptor; and
   (c-2) an expression vector containing a nucleotide sequence encoding an antibody or an antigen binding fragment thereof that specifically binds to a target selected from the group consisting of human leukocyte antigen G and a receptor thereof.

In the expression vectors of (a), (b), and (c) described above, the nucleotide sequence encoding the antibody or antigen binding fragment thereof that specifically binds to the human leukocyte antigen G is identical to the nucleotide sequence described for the immune cell.

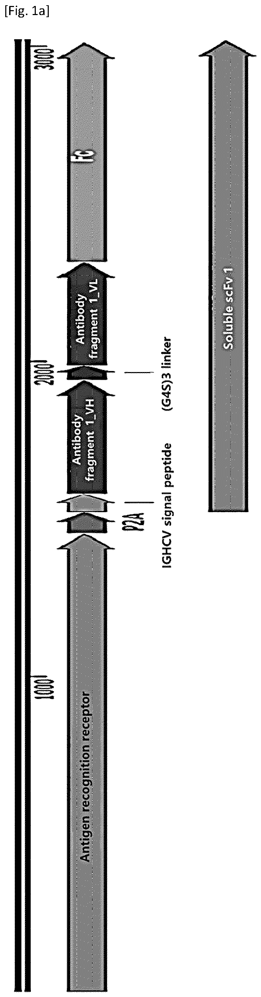

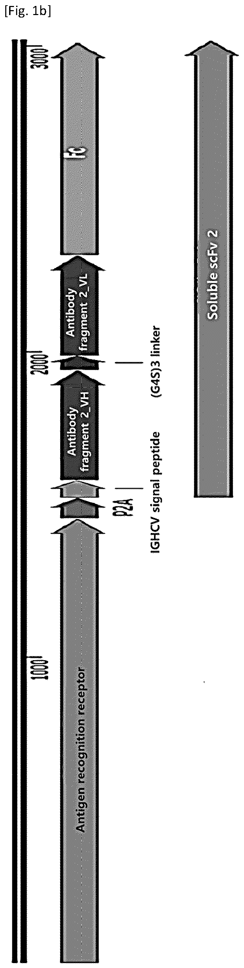

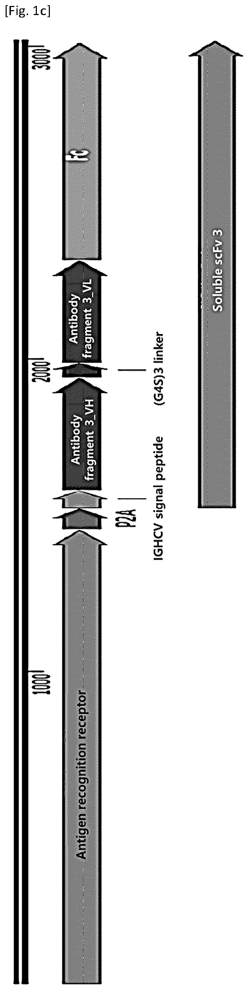

According to one exemplary embodiment of the present invention, the expression vector of (a) may be an expression vector in which a nucleotide sequence encoding an antigen recognition receptor and a nucleotide sequence encoding an antibody or an antigen binding fragment thereof that specifically binds to a target selected from the group consisting of human leukocyte antigen G and a receptor thereof are linked via a nucleotide sequence encoding a self-cleaving peptide (FIGS. 1A to 1C). The nucleotide sequence encoding the self-cleaving peptide is a sequence encoding an amino acid sequence of SEQ ID NO: 7.

According to one exemplary embodiment of the present invention, in the expression vectors of (a), (b), and (c), the nucleotide sequence encoding the antibody or antigen binding fragment thereof that specifically binds to the human leukocyte antigen G (HLA-G) may be specifically a nucleotide sequence obtained by sequentially linking a nucleotide sequence encoding a signal sequence, a nucleotide sequence encoding an anti-HLA-G binding scFv, and a nucleotide sequence encoding a fragment crystallization region (Fc) of human immunoglobulin (FIGS. 1A to 1C).

Also, the nucleotide sequence encoding the anti-HLA-G binding scFv includes a nucleotide sequence encoding a linker sequence between the nucleotide sequences encoding the heavy-chain and light-chain variable regions. In this case, the linker sequence may be a sequence of SEQ ID NO: 13.

According to one exemplary embodiment of the present invention, the signal sequence plays a role in secreting the anti-HLA-G binding scFv expressed in cells by allowing the anti-HLA-G binding scFv to move to the outside of the cells. While existing immune checkpoint inhibitor antibodies such as pembrolizumab (Trade name: Keytruda) are administered into the body as the antibody itself, the present invention may expect the effects of both immune cells and scFv because the anti-HLA-G binding scFv is secreted outside the cells even when immune cells are administered. Also, since the anti-HLA-G binding scFv is expressed and secreted only in the vicinity of cancer cells where immune cells are present, not throughout the body, systemic side effects may be reduced.

The nucleotide sequence encoding the signal sequence may be a nucleotide sequence encoding an amino acid sequence selected from the group consisting of SEQ ID NOS: 8 to 12 or an amino acid sequence having a sequence identity of at least 80%, preferably at least 90%, more preferably at least 95%, and most preferably at least 99% with the amino acid sequence selected from the group consisting of SEQ ID NOS: 8 to 12. In an embodiment of the present invention, the signal sequence of SEQ ID NO: 8 was used.

The Fc region maintains the stability and biological activity of the anti-HLA-G binding scFv, and the nucleotide sequence encoding the Fc region may be a sequence commonly used in the art to which the present invention pertains. In an embodiment of the present invention, the sequence of SEQ ID NO: 14 was used.

In the present invention, the anti-HLA-G binding scFv may also be referred to as anti-HLA-G scFv, antibody fragment scFv, and the like.

In the present invention, the expression vector containing a nucleic acid encoding the soluble scFv that specifically binds to the human leukocyte antigen G may also be referred to as a soluble scFv expression vector.

Various vectors known in the art may be used as the vector used in the present invention, and depending on the type of cells in which the antigen recognition receptor and/or soluble scFv are to be expressed, expression control sequences such as a promoter, a terminator, an enhancer, and the like, sequences for membrane targeting or secretion, and the like can be appropriately selected and combined in various ways according to the purpose.

Methods for introducing the vector of the present invention into cells and expressing the vector of the present invention are well known in the art. The vector may be readily introduced into host cells, such as a mammalian, bacterial, yeast, or insect cells, by methods known in the art. For example, the vector may be delivered into the host cells by physical, chemical, or biological means. The physical means include calcium phosphate precipitation, lipofection, particle bombardment, microinjection, electroporation, and the like. The chemical means include colloidal dispersion systems, such as macromolecular complexes, nanocapsules, microspheres, beads, and lipid-based systems including oil-in-water emulsions, micelles, mixed micelles, and liposomes. Also, the biological means include the use of DNA or RNA vectors, such as the lentiviruses, retroviruses, and the like, as described above.

The vector of the present invention includes a plasmid vector, a cosmid vector, a bacteriophage vector, a viral vector, and the like, but the present invention is not limited thereto. In addition to expression control elements such as a promoter, an operator, an initiation codon, a termination codon, a polyadenylation signal, and an enhancer, suitable vectors include a signal sequence or a leader sequence for membrane targeting or secretion, and may be constructed in various ways depending on the purpose.

In one exemplary embodiment of the present invention, a lentiviral vector was used. In a specific embodiment of the present invention, the vector further includes a promoter. The promoter may be, for example, an RSV promoter, but the present invention is not limited thereto.

Still another aspect of the present invention is an isolated nucleic acid including a nucleotide sequence encoding the anti-HLA-G binding soluble scFv.

As used in the present specification, the term "nucleic acid" is meant to encompass DNA and RNA molecules in a comprehensive manner, and nucleotides, which are the basic structural units in nucleic acid molecules, include not only natural nucleotides but also analogues in which the sugar or base moieties are modified (Scheit, Nucleotide Analogs, John Wiley, New York (1980); Uhlman and Peyman, Chemical Reviews (1990) 90:543-584).

The nucleotide sequence encoding the anti-HLA-G binding scFv of the present invention may be modified, wherein the modifications include additions, deletions, or conservative or non-conservative substitutions of nucleotides.

A nucleic acid (polynucleotide) including a nucleotide sequence encoding the soluble scFv according to the present invention may be modified by codon optimization, which is due to the degeneracy of codons. Therefore, it will be well understood by those skilled in the art that there are many nucleotide sequences encoding a polypeptide or a variant fragment thereof. Some of these nucleic acids have minimal homology to the nucleotide sequence of any naturally occurring gene.

In particular, nucleic acids that are variable due to differences in codon usage, for example, nucleic acids optimized for codon selection in humans, primates and/or mammals are desirable.

In the present invention, the isolated nucleic acid may include the following sequence:
(a) a nucleotide sequence encoding heavy-chain CDR1 including a nucleotide sequence encoding the amino acid sequence of SEQ ID NO: 21, a nucleotide sequence encoding heavy-chain CDR2 including a nucleotide sequence encoding the amino acid sequence of SEQ ID NO: 22, and a nucleotide sequence encoding heavy-chain CDR3 including a nucleotide sequence encoding the amino acid sequence of SEQ ID NO: 23; and
   a nucleotide sequence encoding light-chain CDR1 including a nucleotide sequence encoding the amino acid sequence of SEQ ID NO: 24, a nucleotide sequence encoding light-chain CDR2 including a nucleotide sequence encoding the amino acid sequence of SEQ ID NO: 25, and a nucleotide sequence encoding light-chain CDR3 including a nucleotide sequence encoding the amino acid sequence of SEQ ID NO: 26;
(b) a nucleotide sequence encoding heavy-chain CDR1 including a nucleotide sequence encoding the amino acid sequence of SEQ ID NO: 27, a nucleotide sequence encoding heavy-chain CDR2 including a nucleotide sequence encoding the amino acid sequence of SEQ ID NO: 28, and a nucleotide sequence encoding heavy-chain CDR3 including a nucleotide sequence encoding the amino acid sequence of SEQ ID NO: 29; and
   a nucleotide sequence encoding light-chain CDR1 including a nucleotide sequence encoding the amino acid sequence of SEQ ID NO: 30, a nucleotide sequence encoding light-chain CDR2 including a nucleotide sequence encoding the amino acid sequence of SEQ ID NO: 31, and a nucleotide sequence encoding light-chain CDR3 including a nucleotide sequence encoding the amino acid sequence of SEQ ID NO: 32; or
(c) a nucleotide sequence encoding heavy-chain CDR1 including a nucleotide sequence encoding the amino acid sequence of SEQ ID NO: 33, a nucleotide sequence encoding heavy-chain CDR2 including a nucleotide sequence encoding the amino acid sequence of SEQ ID NO: 34, and a nucleotide sequence encoding heavy-chain CDR3 including a nucleotide sequence encoding the amino acid sequence of SEQ ID NO: 35; and
   a nucleotide sequence encoding light-chain CDR1 including a nucleotide sequence encoding the amino acid sequence of SEQ ID NO: 36, a nucleotide sequence encoding light-chain CDR2 including a nucleotide sequence encoding the amino acid sequence of SEQ ID NO: 37, and a nucleotide sequence encoding light-chain CDR3 including a nucleotide sequence encoding the amino acid sequence of SEQ ID NO: 38.

More specifically, the isolated nucleic acid may include the following sequence:
(a) a nucleotide sequence encoding a heavy-chain variable region having an amino acid sequence of SEQ ID NO: 15 and a nucleotide sequence encoding a light-chain variable region having an amino acid sequence of SEQ ID NO: 16;
(b) a nucleotide sequence encoding a heavy-chain variable region having an amino acid sequence of SEQ ID NO: 17 and a nucleotide sequence encoding a light-chain variable region having an amino acid sequence of SEQ ID NO: 18; or
(c) a nucleotide sequence encoding a heavy-chain variable region having an amino acid sequence of SEQ ID NO: 19 and a nucleotide sequence encoding a light-chain variable region having an amino acid sequence of SEQ ID NO: 20.

Also, according to one exemplary embodiment of the present invention, the isolated nucleic acid further includes a nucleotide sequence encoding a linker sequence of SEQ ID NO: 13 between the nucleotide sequence encoding the heavy-chain variable region and the nucleotide sequence encoding the light-chain variable region.

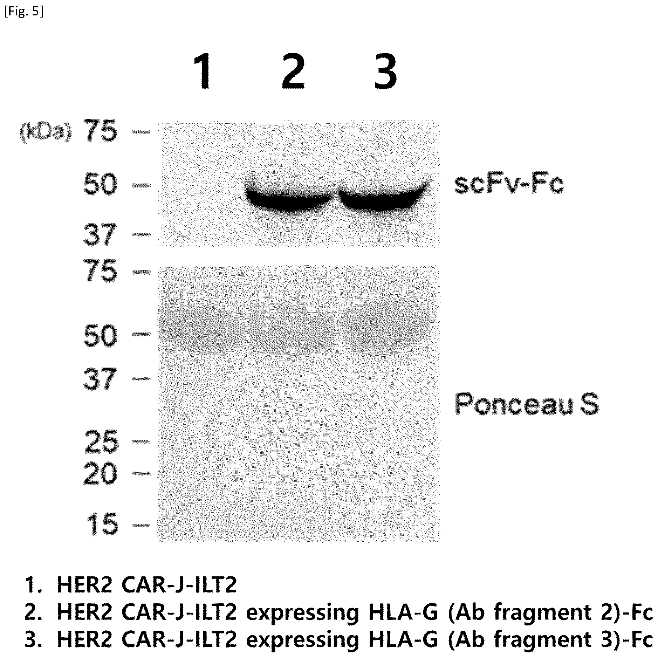

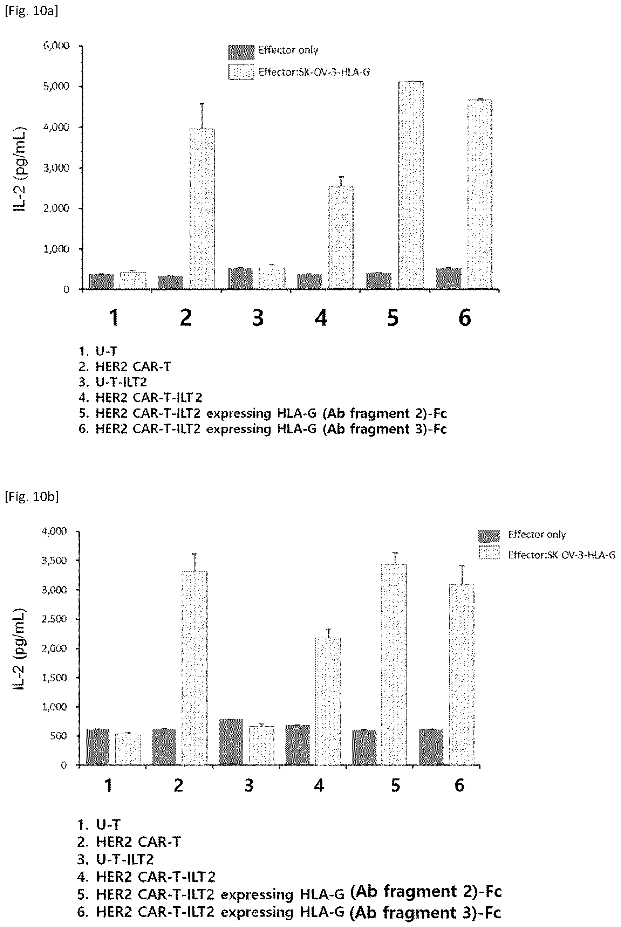

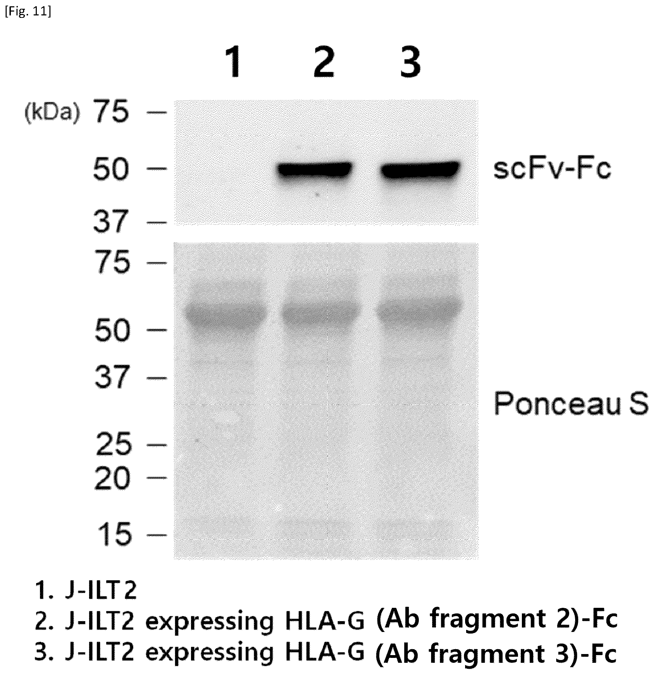

Meanwhile, the present inventors confirmed that soluble scFv is well secreted out of the cells from anti-HLA-G soluble scFv-expressing immune cells (FIGS. 5 and 11), and that HER2 CAR-T cells expressing the soluble scFv do not undergo a decrease in immune cell function due to HLA-G-expressing cancer cell lines (FIG. 10).

That is, the immune cell of the present invention may exhibit an anti-cancer effect by simultaneously expressing a soluble scFv capable of inhibiting the interaction between HLA-G and its receptor together with an antigen recognition receptor. In this case, when the soluble scFv is expressed alone, the soluble scFv may help increase anti-cancer efficacy in the process of recognizing and removing a target (cancer cell) with an endogenous antigen recognition receptor. Since this effect of increasing anti-cancer efficacy may also act on endogenous immune cells such as tumor-infiltrating lymphocytes, it may further induce the activation of endogenous immune cells.

Accordingly, the present invention also provides a pharmaceutical composition including the immune cell of the present invention as described above.

According to one exemplary embodiment of the present invention, the pharmaceutical composition may be used for the prevention or treatment of cancer.

In the present invention, the terms "cancer" and "tumor" are used interchangeably, and refer to or mean a physiological condition in mammals, which is typically characterized by unregulated cell growth/proliferation.

As used in the present specification, the term "prevention" refers to any action that inhibits or delays the progression of a disease by administration of the composition of the present invention, and the term "treatment" refers to any action that inhibits, alleviates, or eliminate the development of a disease.

In the present invention, examples of the cancer (or tumor) include carcinoma, lymphoma (e.g., Hodgkin's and non-Hodgkin's lymphoma), blastoma, sarcoma, and leukemia, but the present invention is not limited thereto. More preferred examples of cancer include pancreatic cancer, breast cancer, ovarian cancer, glioma, cervical cancer, endometrial cancer, esophageal cancer, gastric cancer, liver cancer, lung cancer, colorectal cancer, nasopharyngeal cancer, oral cancer, thyroid cancer, prostate cancer, kidney cancer, melanoma, gallbladder cancer, bile duct cancer, melanoma, squamous cell carcinoma, small-cell lung cancer, non-small-cell lung cancer, pulmonary adenocarcinoma, pulmonary squamous cell carcinoma, peritoneal cancer, hepatocellular cancer, bladder cancer, hepatocellular carcinoma, colon cancer, colorectal cancer, salivary gland carcinoma, vulvar cancer, mesothelioma, leukemia and other lymphoproliferative disorders, and various types of head and neck cancer.

More specifically, the pharmaceutical composition may be used for the treatment of cancer positive for HLA-G expression, and the cancer may be selected from the group consisting of pancreatic cancer, breast cancer, ovarian cancer, glioma, cervical cancer, endometrial cancer, esophageal cancer, gastric cancer, liver cancer, lung cancer, colorectal cancer, nasopharyngeal cancer, oral cancer, thyroid cancer, prostate cancer, kidney cancer, gallbladder cancer, bile duct cancer, and melanoma.

The pharmaceutical composition of the present invention may further include a pharmaceutically acceptable carrier, and the pharmaceutically acceptable carrier is commonly used in the preparation of a formulation, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like, but the present invention is not limited thereto. In addition to the above-described components, the composition for preventing or treating cancer according to the present invention may further include a lubricant, a wetting agent, a sweetening agent, a flavoring agent, an emulsifying agent, a suspending agent, a preservative, and the like. Suitable pharmaceutically acceptable carriers and preparations are described in detail in Remington's Pharmaceutical Sciences (19th ed., 1995).

The pharmaceutical composition of the present invention may be administered orally or parenterally. In the case of parenteral administration, the pharmaceutical composition may be administered by intravenous injection, subcutaneous injection, intramuscular injection, intraperitoneal injection, intradermal administration, topical administration, intranasal administration, intrapulmonary administration, rectal administration, and the like. In the case of oral administration, since proteins or peptides are digested, an oral composition may be formulated to coat an active agent or protect the active agent from decomposition in the stomach, and the composition of the present invention may be administered by any device that allows an active material to travel to target cells.

The appropriate dose of the pharmaceutical composition of the present invention varies depending on factors such as a preparation method, the mode of administration, the age, weight, sex, and pathological condition of a patient, food, an administration time, a route of administration, an excretion rate, and response sensitivity, and a generally skilled physician may easily determine and prescribe a pharmaceutically effective amount effective for the desired treatment or prevention. In the present specification, the term "pharmaceutically effective amount" refers to an amount sufficient for the treatment, prevention, and diagnosis of a disease.

The pharmaceutical composition of the present invention may include, in a single dose, immune cells (e.g., T cells) in an amount that is 1 to 10 times the number of tumor cells, such as pancreatic cancer cells, breast cancer cells, ovarian cancer cells, or glioma cells, in a subject to be treated.

The pharmaceutical composition of the present invention may be prepared in a unit dosage form or it may be prepared by placing it into a multi-dose container by formulating it using a pharmaceutically acceptable carrier and/or excipient according to a method that may be easily performed by a person having ordinary skill in the art to which the present invention pertains, and can be manufactured. In this case, the formulation may be in the form of a solution, suspension or emulsion in an oil or aqueous medium or may be in the form of an extract, powder, suppository, powder, granule, tablet or capsule, and may further include a dispersant or stabilizer.

The pharmaceutical composition of the present invention may be administered as an separate therapeutic agent or in combination with other therapeutic agents, and may be administered sequentially or simultaneously with conventional therapeutic agents.

Yet another aspect of the present invention provides a use of the immune cell according to any one of claims 1 to 10 for the preparation of a pharmaceutical composition for preventing or treating cancer, and a use of the immune cell according to any one of claims 1 to 10 for preventing or treating cancer.

The present invention also provides a method for preventing or treating cancer, which includes administering the above-described immune cell or pharmaceutical composition to a subject in need thereof.

The subject refers to any animal, including humans as well as monkeys, cows, horses, sheep, pigs, chickens, turkeys, quails, cats, dogs, mice, rats, rabbits, or guinea pigs that have developed or may develop cancer.

The cancer may be a cancer in which HLA-G is overexpressed, but the present invention is not limited thereto.

### [Advantageous Effects]

The present invention provides an immune cell that overcomes an immune checkpoint and a pharmaceutical composition using the same. HLA-G acts as an immune checkpoint in various solid cancers to suppresses the normal function of tumor-infiltrating lymphocytes. However, since the immune cell according to the present invention expresses and secretes soluble scFv derived from an anti-HLA-G antibody, the immune cell can overcome the immunosuppressive response to eliminate cancer cells more efficiently and further induce the activation of endogenous immune cells. Therefore, the immune cell according to the present invention can be used for the treatment of various cancers associated with high expression of HLA-G.

### [Description of Drawings]

FIGS. 1A to 1C show the structures of an antigen recognition receptor construct expressing soluble scFv 1, an antigen recognition receptor construct expressing soluble scFv 2, and an antigen recognition receptor construct expressing soluble scFv 3 according to embodiments of the present invention, respectively.
FIGS. 2A to 2C show the structures of the pLV-EF1A-antigen recognition receptor-soluble scFv 1, the pLV-EF1A-antigen recognition receptor-soluble scFv 2, and the pLV-EF1A-antigen recognition receptor-soluble scFv 3 plasmids of the present invention, respectively.
FIGS. 3A to 3C show the structures of the pLV-EF1A-soluble scFv 1, the pLV-EF1A-soluble scFv 2, and the pLV-EF1A-soluble scFv 3 plasmids of the present invention, respectively.
FIG. 4 shows the result of confirming the expression levels of CAR and ILT2 on the surface of anti-HER2 CAR-expressing Jurkat cells secreting the soluble scFv of the present invention.
FIG. 5 shows the result of confirming that soluble scFv is normally secreted from the anti-HER2 CAR-expressing Jurkat cells secreting the soluble scFv of the present invention.
FIG. 6 shows the result of confirming the activity of the anti-HER2 CAR-expressing Jurkat cells secreting the soluble scFv of the present invention against the SK-OV-3-HLA-G cell line through secretion of IL-2.
FIG. 7 shows the result of confirming the activity of the anti-HER2 CAR-expressing Jurkat cells secreting the soluble scFv of the present invention against the SK-OV-3-HLA-G cell line through activation of CD25.
FIG. 8 shows the result of confirming the expression levels of CAR and ILT2 on the surface of anti-HER2 CAR-expressing T cells secreting the soluble scFv of the present invention.
FIG. 9 shows the result of confirming that soluble scFv is normally secreted from the anti-HER2 CAR-expressing T cells secreting the soluble scFv of the present invention.
FIGS. 10A and 10B show the activity of the anti-HER2 CAR-expressing T cells secreting the soluble scFv of the present invention against the SK-OV-3-HLA-G cell line through secretion of IL-2.
FIG. 119 shows the result of confirming that soluble scFv is normally secreted from Jurkat cells secreting the soluble scFv of the present invention.

### [Mode for Invention]

Hereinafter, the present invention will be described in more detail through examples. However, it will be apparent to those skilled in the art that these examples are merely provided to illustrate the present invention, and are not intended to limit the scope of the present invention.

### Example 1: Cell line and culture

A human ovarian cancer cell line SK-OV-3 and a human acute leukemia cell line Jurkat were provided by the Korea Cell Line Bank (Korea), and used. A SK-OV-3-HLA-G cell line, which stably overexpresses HLA-G, and a Jurkat-ILT2 cell line, which stably overexpresses an HLA-G receptor, Ig-like transcript 2 (ILT2), were produced from the SK-OV-3 or Jurkat cell lines, respectively, using a lentiviral vector. The SK-OV-3-HLA-G and Jurkat-ILT2 cell lines were cultured in an RPMI 1640 (Gibco, USA) medium supplanted with 10% FBS (Gibco, USA), 1% antibiotics (antibiotic-antimycotic; Gibco, USA), and 0.5 ug/mL of puromycin (Sigma-Aldrich, USA).

### Example 2: Construction of anti-HLA-G soluble scFv construct

A construct expressing the antigen recognition receptor and the anti-HLA-G soluble scFv of the present invention was artificially synthesized. More specifically, the antigen recognition receptor and the soluble scFv were linked by a self-cleaving peptide, and the soluble scFv was artificially synthesized by splicing by overlap extension PCR (SOE-PCR) in a state in which a human immunoglobulin heavy-chain signal sequence, an HLA-G antibody fragment scFv, and a human immunoglobulin Fc region were linked (FIGS. 1A to 1C).

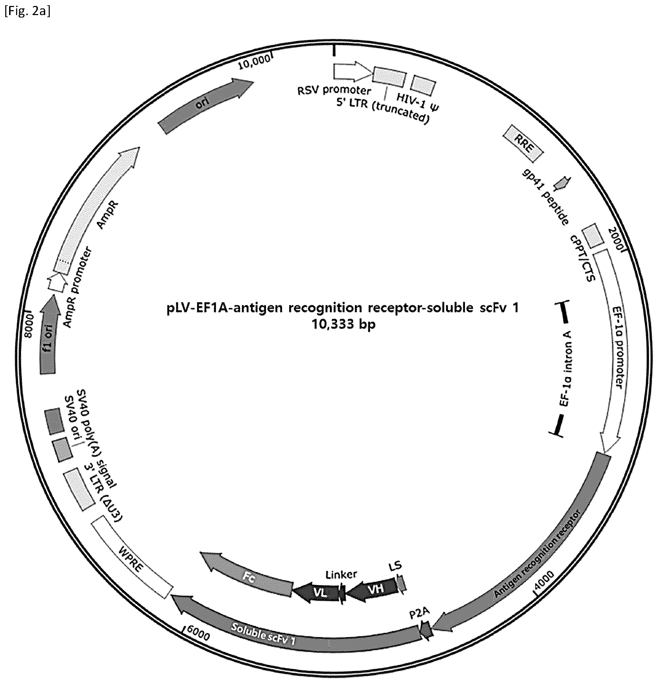

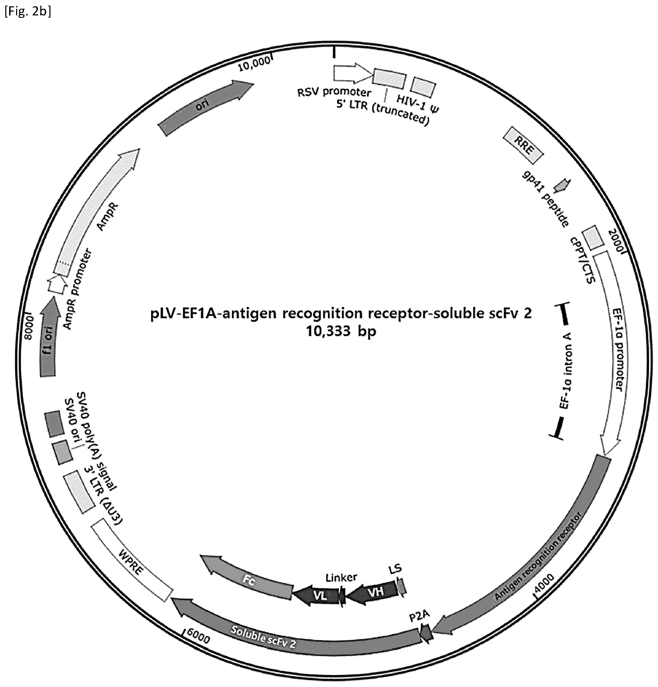

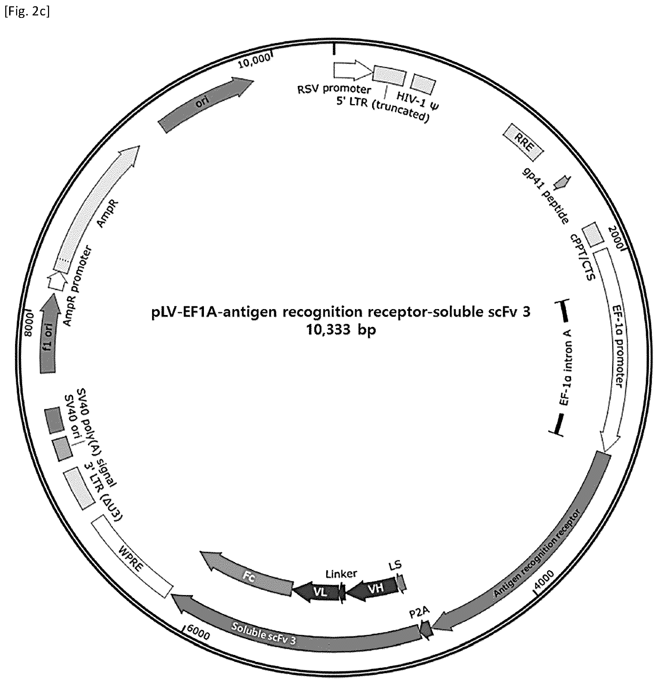

The PCR products were digested with Mlu I and Xba I, and then inserted into the Mlu I and Xba I sites of the pLV-EF1A-MCS vector, which is a third-generation self-inactivating lentiviral expression vector (FIGS. 2A to 2C).

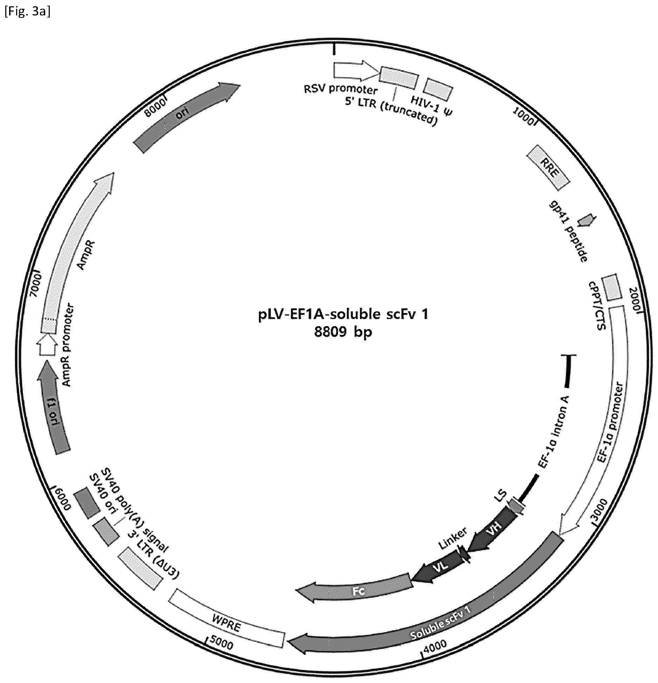

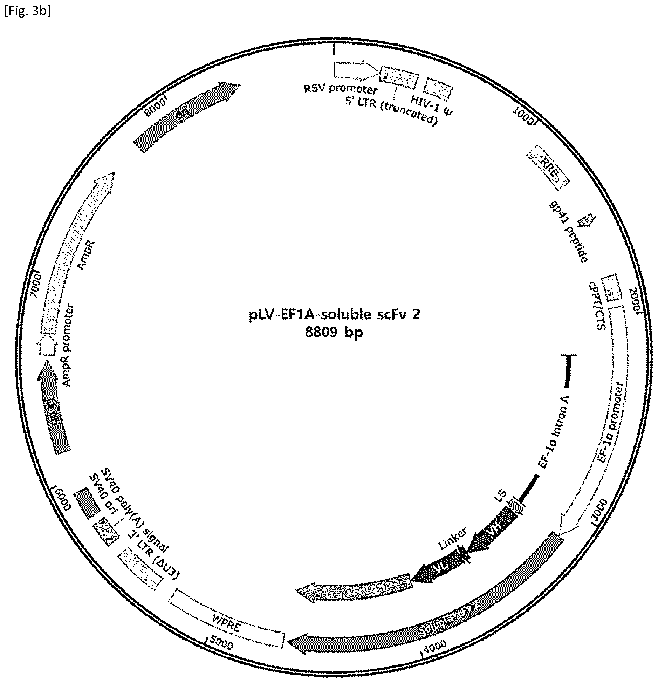

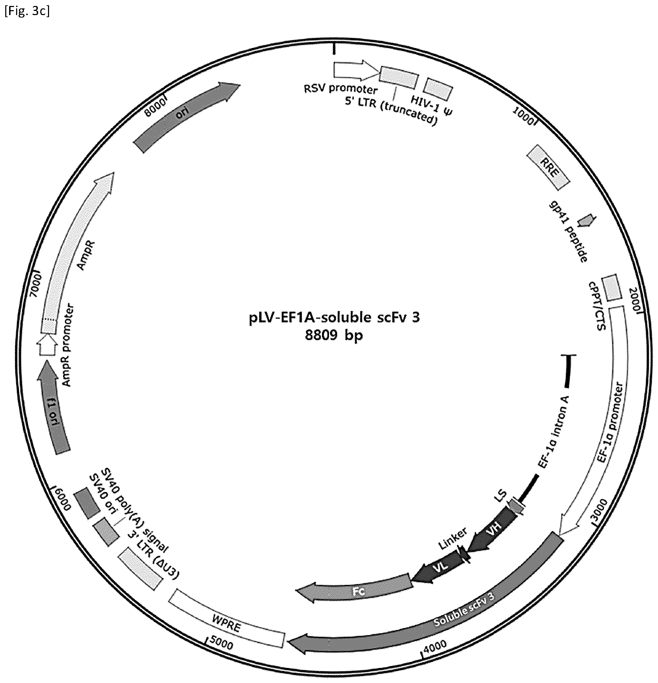

For immune cells using endogenous antigen recognition receptors, a construct expressing only the soluble scFv without any antigen recognition receptor was also constructed. The soluble scFv was artificially synthesized by SOE-PCR in a state in which the human immunoglobulin heavy-chain signal sequence, the HLA-G antibody fragment scFv, and the human immunoglobulin Fc region were linked, and the PCR products were digested with Mlu I and Xba I, and then inserted into the Mlu I and Xba I sites of the pLV-EF1A-MCS vector, which is a third-generation self-inactivating lentiviral expression vector (FIGS. 3A to 3C).

Information on the sequences of the anti-HLA-G soluble scFv according to one embodiment of the present invention and domains used in the preparation of the anti-HLA-G soluble scFv is summarized in Table 1. Each region constituting the soluble scFv is connected in tandem with each other, and also connected in frame. Specifically, a signal sequence, an antibody fragment scFv, an Fc region, and a stop codon (*i.e.,* TAA), are connected.

**[Table 1]**

| SEQ ID NO | Sequence Name | Sequence |
|---|---|---|
| 1 | Antibody fragment 1 scFv amino acid | EVQLLESGGGLVQPGGSLRLSCAASGFTFSDYAMSWV RQAPGKGLEWVSVISHDGGSIYYADSVKGRFTISRDNS |
| | | KNTLYLQMNSLRAEDTAVYYCAKGPRRIANAIFDYWG QGTLVTVSSGGGGSGGGGSGGGGSQFVLTQPPSVSAAP GQKVTISCSGSNSNIGNSYVSWYQQVPGAAPRLLIYGST NRPSGVPDRFSGSKSGTSASLAITGLQAEDEADYYCQS YDSSLSGYVFGTGTKVTVL |
| 2 | Antibody fragment 2 scFv amino acid | EVQLLESGGGLVQPGGSLRLSCAASGFTFSDYAMSWV RQAPGKGLEWVSVISHDGDRVYYADSVKGRFTISRDNS KNTLYLQMNSLRAEDTAVYYCAKGPRRIANAIFDYWG QGTLVTVSSGGGGSGGGGSGGGGSQLVLTQPPSVSGAP GQKVTISCSGSSSNIGHSYVSWYQQLPGTAPKLLIYGDN NRPSGVPDRFSGSKSGTSASLAITGLQAEDEADYYCQS YDSSLSGYVFGTGTKVTVL |
| 3 | Antibody fragment 3 scFv amino acid | EVQLLESGGGLVQPGGSLRLSCAASGFTFSDYSMSWVR QAPGKGLEWVSAISPDRSLEYYADSVKGRFTISRDNSK NTLYLQMNSLRAEDTAVYYCAKGPRHLTNNIFDYWGQ GTLVTVSSGGGGSGGGGSGGGGSQFVLTQPPSVSAAPG QKVTISCSGSSSNIGHSYVSWYQQLPGTAPKLLIYGNIH RPSGVPDRFSGSTSGTSASLAITGLQADDEADYYCGVW DSSLSAVVFGGGTKLTVL |
| 4 | Soluble scFv 1 amino acid | MEFGLSWLFLVAILKGVQCEVQLLESGGGLVQPGGSLR LSCAASGFTFSDYAMSWVRQAPGKGLEWVSVISHDGG SIYYADSVKGRFTISRDNSKNTLYLQMNSLRAEDTAVY YCAKGPRRIANAIFDYWGQGTLVTVSSGGGGSGGGGS GGGGSQFVLTQPPSVSAAPGQKVTISCSGSNSNIGNSYV SWYQQVPGAAPRLLIYGSTNRPSGVPDRFSGSKSGTSA SLAITGLQAEDEADYYCQSYDSSLSGYVFGTGTKVTVL EPKSCDKTHTCPPCPAPELLGGPSVFLFPPKPKDTLMISR TPEVTCVVVDVSHEDPEVKFNWYVDGVEVHNAKTKP |
| | | REEQYNSTYRVVSVLTVLHQDWLNGKEYKCKVSNKA LPAPIEKTISKAKGQPREPQVYTLPPSREEMTKNQVSLT CLVKGFYPSDIAVEWESNGQPENNYKTTPPVLDSDGSF FLYSKLTVDKSRWQQGNVFSCSVMHEALHNHYTQKSL SLSPGK |
| 5 | Soluble scFv 2 amino acid | MEFGLSWLFLVAILKGVQCEVQLLESGGGLVQPGGSLR LSCAASGFTFSDYAMSWVRQAPGKGLEWVSVISHDGD RVYYADSVKGRFTISRDNSKNTLYLQMNSLRAEDTAV YYCAKGPRRIANAIFDYWGQGTLVTVSSGGGGSGGGG SGGGGSQLVLTQPPSVSGAPGQKVTISCSGSSSNIGHSY VSWYQQLPGTAPKLLIYGDNNRPSGVPDRFSGSKSGTS ASLAITGLQAEDEADYYCQSYDSSLSGYVFGTGTKVTV LEPKSCDKTHTCPPCPAPELLGGPSVFLFPPKPKDTLMIS RTPEVTCVVVDVSHEDPEVKFNWYVDGVEVHNAKTK PREEQYNSTYRVVSVLTVLHQDWLNGKEYKCKVSNK ALPAPIEKTISKAKGQPREPQVYTLPPSREEMTKNQVSL TCLVKGFYPSDIAVEWESNGQPENNYKTTPPVLDSDGS FFLYSKLTVDKSRWQQGNVFSCSVMHEALHNHYTQKS LSLSPGK |
| 6 | Soluble scFv 3 amino acid | MEFGLSWLFLVAILKGVQCEVQLLESGGGLVQPGGSLR LSCAASGFTFSDYSMSWVRQAPGKGLEWVSAISPDRSL EYYADSVKGRFTISRDNSKNTLYLQMNSLRAEDTAVY YCAKGPRHLTNNIFDYWGQGTLVTVSSGGGGSGGGGS GGGGSQFVLTQPPSVSAAPGQKVTISCSGSSSNIGHSYV SWYQQLPGTAPKLLIYGNIHRPSGVPDRFSGSTSGTSAS LAITGLQADDEADYYCGVWDSSLSAVVFGGGTKLTVL EPKSCDKTHTCPPCPAPELLGGPSVFLFPPKPKDTLMISR TPEVTCVVVDVSHEDPEVKFNWYVDGVEVHNAKTKP |
| | | REEQYNSTYRVVSVLTVLHQDWLNGKEYKCKVSNKA LPAPIEKTISKAKGQPREPQVYTLPPSREEMTKNQVSLT CLVKGFYPSDIAVEWESNGQPENNYKTTPPVLDSDGSF FLYSKLTVDKSRWQQGNVFSCSVMHEALHNHYTQKSL SLSPGK |
| 7 | P2A amino acid | GSGATNFSLLKQAGDVEENPGP |
| 8 | Human immunoglobulin heavy-chain signal sequence amino acid | MEFGLSWLFLVAILKGVQC |
| 9 | Mouse Ig kappa light-chain precursor signal sequence amino acid | MDMRAPAGIFGFLLVLFPGYRS |
| 10 | Human serum albumin preproprotein signal sequence amino acid | MKWVTFISLLFLFSSAYS |
| 11 | Human Azurocidin preproprotein signal sequence amino acid | MTRLTVLALLAGLLASSRA |
| 12 | Human CD8 signal sequence amino acid | MALPVTALLLPLALLLHAARP |
| 13 | Linker amino acid | GGGGSGGGGSGGGGS |
| 14 | Human Imunoglobulin Fc amino acid | EPKSCDKTHTCPPCPAPELLGGPSVFLFPPKPKDTLMISR TPEVTCVVVDVSHEDPEVKFNWYVDGVEVHNAKTKP REEQYNSTYRVVSVLTVLHQDWLNGKEYKCKVSNKA LPAPIEKTISKAKGQPREPQVYTLPPSREEMTKNQVSLT CLVKGFYPSDIAVEWESNGQPENNYKTTPPVLDSDGSF FLYSKLTVDKSRWQQGNVFSCSVMHEALHNHYTQKSL SLSPGK |
| 15 | Antibody fragment 1 scFv heavy-chain variable region amino acid | EVQLLESGGGLVQPGGSLRLSCAASGFTFSDYAMSWV RQAPGKGLEWVSVISHDGGSIYYADSVKGRFTISRDNS KNTLYLQMNSLRAEDTAVYYCAKGPRRIANAIFDYWG QGTLVTVSS |
| 16 | Antibody fragment 1 scFv light-chain variable region amino acid | QFVLTQPPSVSAAPGQKVTISCSGSNSNIGNSYVSWYQ QVPGAAPRLLIYGSTNRPSGVPDRFSGSKSGTSASLAIT GLQAEDEADYYCQSYDSSLSGYVFGTGTKVTVL |
| 17 | Antibody fragment 2 scFv heavy-chain variable region amino acid | EVQLLESGGGLVQPGGSLRLSCAASGFTFSDYAMSWV RQAPGKGLEWVSVISHDGDRVYYADSVKGRFTISRDNS KNTLYLQMNSLRAEDTAVYYCAKGPRRIANAIFDYWG QGTLVTVSS |
| 18 | Antibody fragment 2 scFv light-chain variable region amino acid | QLVLTQPPSVSGAPGQKVTISCSGSSSNIGHSYVSWYQQ LPGTAPKLLIYGDNNRPSGVPDRFSGSKSGTSASLAITG LQAEDEADYYCQSYDSSLSGYVFGTGTKVTVL |
| 19 | Antibody fragment 3 scFv heavy-chain variable region amino acid | EVQLLESGGGLVQPGGSLRLSCAASGFTFSDYSMSWVR QAPGKGLEWVSAISPDRSLEYYADSVKGRFTISRDNSK NTLYLQMNSLRAEDTAVYYCAKGPRHLTNNIFDYWGQ GTLVTVSS |
| 20 | Antibody fragment 3 scFv light-chain Variable region amino acid | QFVLTQPPSVSAAPGQKVTISCSGSSSNIGHSYVSWYQQ LPGTAPKLLIYGNIHRPSGVPDRFSGSTSGTSASLAITGL QADDEADYYCGVWDSSLSAVVFGGGTKLTVL |
| 21 | Antibody fragment 1 scFv heavy-chain variable region CDR1 amino acid | DYAMS |
| 22 | Antibody fragment 1 scFv heavy-chain variable region CDR2 amino acid | VISHDGGSIYYADSVKG |
| 23 | Antibody fragment 1 scFv heavy-chain variable region CDR3 amino acid | GPRRIANAIFDY |
| 24 | Antibody fragment 1 scFv light-chain variable region CDR1 amino acid | SGSNSNIGNSYVS |
| 25 | Antibody fragment 1 scFv light-chain variable region CDR2 amino acid | GSTNRPS |
| 26 | Antibody fragment 1 scFv light-chain variable region CDR3 amino acid | QSYDSSLSGYV |
| 27 | Antibody fragment 2 scFv heavy-chain variable region CDR1 amino acid | DYAMS |
| 28 | Antibody fragment 2 scFv heavy-chain variable region CDR2 amino acid | VISHDGDRVYYADSVKG |
| 29 | Antibody fragment 2 scFv heavy-chain variable region CDR3 amino acid | GPRRIANAIFDY |
| 30 | Antibody fragment 2 scFv light-chain variable region CDR1 amino acid | SGSSSNIGHSYVS |
| 31 | Antibody fragment 2 scFv light-chain variable region CDR2 amino acid | GDNNRPS |
| 32 | Antibody fragment 2 scFv light-chain variable region CDR3 amino acid | QSYDSSLSGYV |
| 33 | Antibody fragment 3 scFv heavy-chain variable region CDR1 amino acid | DYSMS |
| 34 | Antibody fragment 3 scFv heavy-chain variable region CDR2 amino acid | AISPDRSLEYYADSVKG |
| 35 | Antibody fragment 3 scFv heavy-chain variable region CDR3 amino acid | GPRHL TNNIFDY |
| 36 | Antibody fragment 3 scFv light-chain variable region CDR1 amino acid | SGSSSNIGHSYVS |
| 37 | Antibody fragment 3 scFv light-chain variable region CDR2 amino acid | GNIHRPS |
| 38 | Antibody fragment 3 scFv light-chain variable region CDR3 amino acid | GVWDSSLSAVV |

### Example 3: Production of CAR-Jurkat cell line expressing anti-HLA-G soluble scFv

### 3-1: Preparation of anti-HLA-G soluble scFv gene-expressing lentivirus

HER2 CAR was selected as an example of an antigen recognition receptor. HER2 CAR and anti-HLA-G soluble scFv were allowed to be simultaneously expressed through a self-cleaving peptide, and lentiviruses for the delivery of these genes were produced by a plasmid DNA transfection method. A TransIT-293 transfection reagent (Mirus Bio LLC, USA) was used, and this process was performed according to the manufacturer's protocol. On the day before the experiment, a Lenti-X^{™} 293T cell line (Clontech, USA) was seeded at a density of 5 × 10⁶ cells in a 100 mm dish. The next day, the cells were transfected with a pLV-EF1A-antigen recognition receptor-soluble scFv lentiviral vector, a gag-pol expression vector, and a VSV-G envelope expression vector. The antigen recognition receptor-soluble scFv lentiviral vector is as follows: HER2 CAR, HER2 CAR-soluble scFv 2, or HER CAR-soluble scFv 3. After transfection, the cells were cultured for approximately 72 hours, and all cell supernatants were harvested when the culture was completed. The supernatants were filtered through a 0.45 um filter (Millipore, USA) to remove cell debris. The lentivirus was concentrated approximately 100-fold using a Lenti-X^{™} concentrator (Clontech, USA) according to the manufacturer's protocol, and stored at -80°C in a freezer until use.

### 3-2: Production and culture of CAR-Jurkat cell line expressing anti-HLA-G soluble scFv

A Jurkat-ILT2 cell line was seeded in a 6-well plate at a density of 1 × 10⁶ cells per well along with a lentivirus having a multiplicity of infection (MOI) of approximately 5, and the culture medium was adjusted to 2 mL. Also, polybrene was added to the medium at a concentration of 8 ug/mL. After static culture for 24 hours, the medium was replaced with a fresh medium. The transduced Jurkat-ILT2 cell line was maintained by subculturing at 2 × 10⁵ cells per mL at intervals of 2 to 3 days.

### 3-3: Confirmation of secretion of soluble scFv in anti-HLA-G soluble scFv-expressing CAR-Jurkat cell line

The present inventors confirmed whether HER CAR was expressed on the cell surface in the previously produced anti-HLA-G soluble scFv-expressing CAR-Jurkat cell line. Since HER2 CAR and soluble scFv are linked by a self-cleaving peptide, it can be predicted that the soluble scFv will also be expressed normally when CAR is expressed normally.

After 1 × 10⁵ cells were harvested, the cells were suspended in 100 uL of D-PBS to prepare a cell suspension. Recombinant Protein L (PE conjugated) (Sino Biological, China) and a human ILT2 APC-conjugated antibody (R&D Systems, USA) were added to the cell sample and allowed to react at 4°C for 30 minutes. After the reaction was completed, the cells were washed twice with D-PBS, and the expression levels of HER2 CAR and ILT2 were determined by flow cytometry.

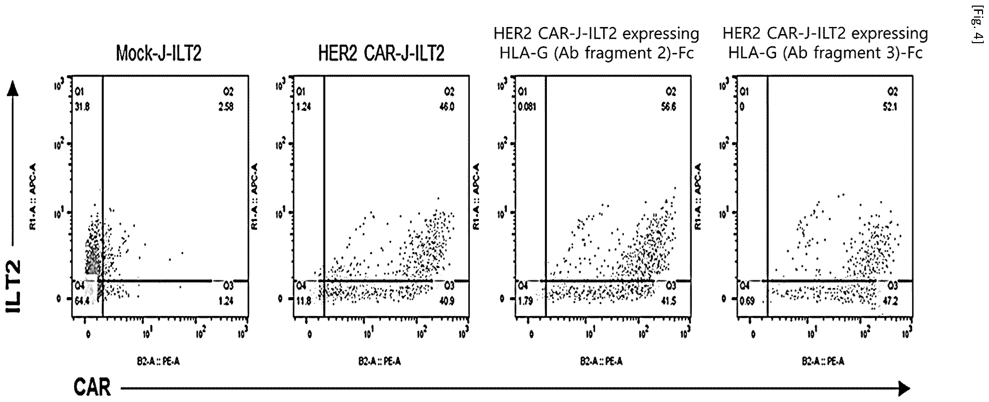

As a result, in the CAR-Jurkat cell line expressing the soluble scFv, almost all the cells expressed HER2 CAR, and more than half of these cells simultaneously expressed ILT2 (FIG. 4).

Since the soluble scFv has to be secreted out of cell to act, it was confirmed whether the soluble scFv was normally expressed and secreted out of the cells. 8 × 10⁵ cells were seeded in a 24-well plate, and all the media was harvested the next day. Then, the protein in the media was obtained using a Minute^{™} High-Efficiency Protein Precipitation kit (Invent Biotechnologies, USA). The protein obtained through Western blotting was detected using a goat anti-human IgG antibody, Fc, HRP conjugate (Sigma-Aldrich, USA).

As a result, it was confirmed that the soluble scFv was well secreted and stably present in the CAR-Jurkat cell line expressing the soluble scFv (FIG. 5).

### Example 4: In vitro confirmation of anti-cancer efficacy of anti-HLA-G soluble scFv-expressing CAR-Jurkat cell line

To confirm the anti-cancer efficacy of the anti-HLA-G soluble scFv-expressing CAR-Jurkat cell line, an SK-OV-3-HLA-G cell line was used. Since the SK-OV-3-HLA-G cell line is a cell line that is positive for both HER2 and HLA-G, HER2 CAR may cause immune cells to exhibit anti-cancer efficacy against target cell lines. Conversely, HLA-G may suppress the anti-cancer efficacy of immune cells against target cell lines. The anti-HLA-G soluble scFv-expressing CAR-Jurkat or control Jurkat cell line was added to a 48-well plate at a density of 2 × 10⁵ cells/150 uL per well, and the SK-OV-3-HLA-G cell line was then added at a density of 2 × 10⁵ cells/150 uL and allowed to react for 48 hours.

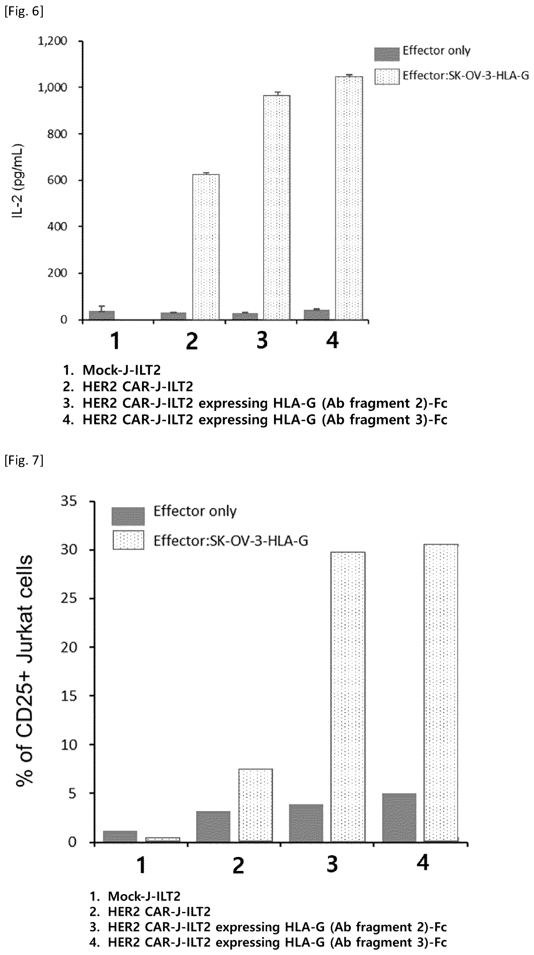

The cell supernatant was harvested, and the concentration of IL-2 in the harvested supernatant was measured using a human IL-2 Quantikine ELISA kit (R&D Systems, USA). As a result, it was confirmed that IL-2 secretion significantly increased when the HER2 CAR-Jurkat cell line expressing the anti-HLA-G soluble scFv was co-cultured with the target cell line, compared to the Jurkat cell line expressing only the HER2 CAR (FIG. 6).

At the same time, the co-cultured cells were harvested, and CD25, an activation marker of the Jurkat cell line, was analyzed by flow cytometry using a CD25 antibody, anti-human, PE-Vio^{®} 770, REAfinity^{™} (Miltenyi Biotec, Germany). As a result, it was confirmed that the cell surface expression level of CD25 significantly increased only when the HER2 CAR-Jurkat cell line expressing the anti-HLA-G soluble scFv was co-cultured with the target cell line, compared to the Jurkat cell line expressing only the HER2 CAR (FIG. 7).

Based on the above results, it can be seen that the decreased function of immune cells due to HLA-G expressed by the target cell line was partially restored when the anti-HLA-G soluble scFv was expressed and secreted.

### Example 5: Production of CAR-T cells expressing anti-HLA-G soluble scFv

### 5-1: Preparation of ILT2 gene-expressing lentivirus and anti-HLA-G soluble scFv gene-expressing lentivirus

Lentiviruses for delivery of the ILT2 gene and anti-HLA-G soluble scFv gene were produced by a plasmid DNA transfection method. A TransIT-293 transfection reagent (Mirus Bio LLC, USA) was used, and this process was performed according to the manufacturer's protocol. On the day before the experiment, Lenti-X^{™} 293T cells (Clontech, USA) were seeded at a density of 5 × 10⁶ in a 100 mm dish. The next day, the cells were transfected with a pLVX-puro-ILT2 lentiviral vector or pLV-EF1A-antigen recognition receptor-soluble scFv lentiviral vector, a gag-pol expression vector, and a VSV-G envelope expression vector. The antigen recognition receptor-soluble scFv lentiviral vector is as follows: HER2 CAR, HER2 CAR-soluble scFv 2, or HER CAR-soluble scFv 3. After transfection, the cells were cultured for approximately 72 hours, and all cell supernatants were harvested after the culture was completed. The supernatants were filtered through a 0.45 um filter (Millipore, USA) to remove cell debris. The lentivirus was concentrated approximately 100-fold using the Lenti-X^{™} concentrator (Clontech, USA) according to the manufacturer's protocol, and stored at -80°C in a freezer until use.

### 5-2: Production and culture of anti-HLA-G soluble scFv-expressing CAR-T cells

Donors were recruited, and leukocytes were obtained through leukapheresis. Peripheral blood mononuclear cells (PBMCs) were obtained from the leukocytes using SepMate^{™}-50 (STEMCELL Technology, Canada) and Ficoll-Paque PLUS (GE Healthcare, Sweden). Thereafter, human T cells were separated by a positive selection method using a QuadroMACS^{™} separator (Miltenyi Biotec, Germany), an LS column (Miltenyi Biotec, Germany), human CD4 MicroBeads (Miltenyi Biotec, Germany), and human CD8 MicroBeads (Miltenyi Biotec, Germany). The human T cells were activated by adding T Cell TransAct^{™}, human (Miltenyi Biotec, Germany) using a TexMACS^{™} medium (Miltenyi Biotec, Germany) as the culture medium. For the growth of T cells, human IL-2 (R&D Systems, USA) was added to the culture medium at a concentration of 100 U/mL and cultured. After 24 hours of culture, the activated T cells were harvested and used for lentiviral delivery.

To deliver both the ILT2 gene and the anti-HLA-G soluble scFv gene, gene transduction was performed at an interval of one day, and RetroNectin^{®} (Takara, Japan) was used to enhance transduction efficiency. The ILT2 gene-expressing lentivirus with an MOI of approximately 10 was put into a 24-well plate coated with RetroNectin, and then adsorbed according to the manufacturer's protocol. Thereafter, 5 × 10⁵ activated human T cells were added per well to the lentivirus-adsorbed plate, and the culture medium was adjusted to 1.25 mL. Then, the ILT2 gene-expressing lentivirus was delivered to the T cells by centrifugation at 1,000 × g for 15 minutes. 24 hours later, the anti-HLA-G soluble scFv gene-expressing lentivirus was delivered to the T cells in the same manner. Then, the T cells were statically cultured for 48 hours, and the medium was then replaced with a fresh medium. The transduced human T cells were subcultured at 2 × 10⁵ cells per mL at intervals of 2 to 3 days, and maintained so that the cell number did not exceed 2 × 10⁶ cells per mL. Unless otherwise specified, the human T cells were cultured in a culture medium supplemented with 100 U/mL of IL-2 (R&D Systems, USA).

### 5-3: Confirmation of secretion of soluble scFv from anti-HLA-G soluble scFv-expressing CAR-T cells

It was confirmed whether HER CAR was expressed on the cell surface in the previously produced anti-HLA-G soluble scFv-expressing CAR-T cells. Since the HER2 CAR and soluble scFv are linked via a self-cleaving peptide, it can be predicted that when CAR is expressed normally, soluble scFv will also be expressed normally.

After 1 × 10⁵ cells were harvested, the cells were suspended in 100 uL of D-PBS to prepare a cell suspension. Recombinant Protein L (PE conjugated) (Sino Biological, China) and a human ILT2 APC-conjugated antibody (R&D Systems, USA) were added to the cell sample and allowed to react at 4°C for 30 minutes. After the reaction was completed, the cells were washed twice with D-PBS, and the expression levels of HER2 CAR and ILT2 were determined by flow cytometry.

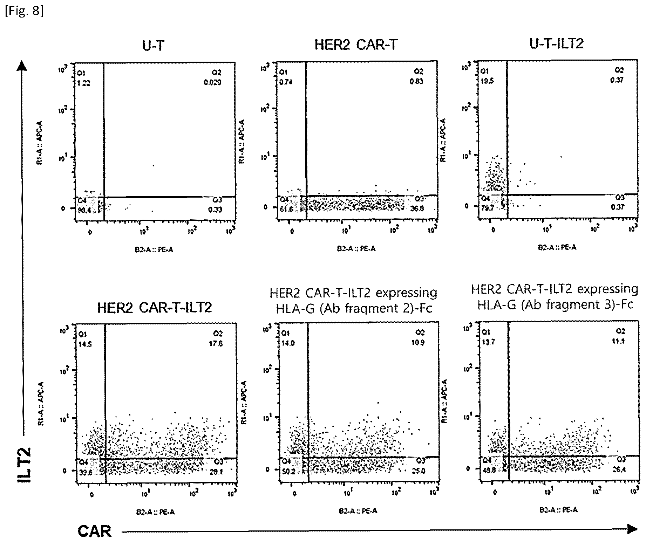

As a result, the CAR expression level was in the range of 35 to 45% in the HER2 CAR-transduced cells, and the ILT2 expression level was in the range of 20 to 30% in the ILT2-transduced cells (FIG. 8).

Since the soluble scFv has to be secreted out of cells to act, it was confirmed whether the soluble scFv was normally expressed and secreted out of the cells. 8 × 10⁵ cells were seeded in a 24-well plate. The next day, all the media was harvested, and the protein in the media was obtained using a Minute^{™} High-Efficiency Protein Precipitation kit (Invent Biotechnologies, USA). The protein obtained through Western blotting was detected using a goat anti-human IgG antibody, Fc, HRP conjugate (Sigma-Aldrich, USA).

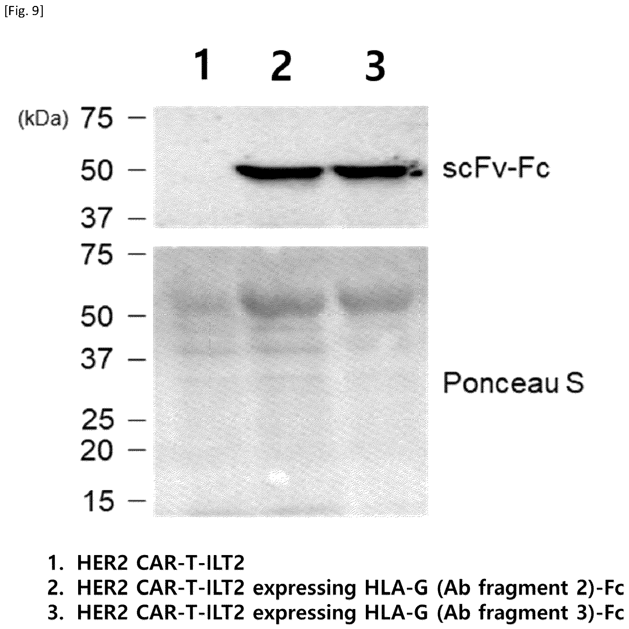

As a result, it was confirmed that the soluble scFv was well secreted and stably present in the CAR-T cells expressing the soluble scFv (FIG. 9).

### Example 6: In vitro confirmation of anti-cancer efficacy of anti-HLA-G soluble scFv-expressing CAR-T cells

To confirm the anticancer efficacy of the anti-HLA-G soluble scFv-expressing CAR-T cells, an SK-OV-3-HLA-G cell line was used. Since the SK-OV-3-HLA-G cell line is a cell line that is positive for both HER2 and HLA-G, HER2 CAR may cause immune cells to exhibit anti-cancer efficacy against target cell lines. Conversely, HLA-G may suppress the anti-cancer efficacy of immune cells against target cell lines. The SK-OV-3-HLA-G cell line (target cells = T) was added to a 96-well plate at a density of 1 × 10⁴ cells/150 uL per well. 24 hours later, the anti-HLA-G soluble scFv-expressing CAR-T or control T cells (effector cells = E) were added at a density of 5 × 10⁴ cells/70 uL or 3 × 10⁴ cells/70 uL per well (E:T ratio = 5:1 or 3:1), and then allowed to react for 48 hours.

The cell supernatants were harvested, and the concentration of IL-2 in the harvested supernatants was measured using a Human IL-2 Quantikine ELISA kit (R&D Systems, USA). As a result, it was confirmed that in the case of both E:T ratios of 5 and 3, the IL-2 secretion of the HER2 CAR-T-ILT2 cells further expressing ILT2 decreased compared to the CAR-T cells expressing only HER2 CAR, and this decrease was significantly restored in the HER2 CAR-T cells expressing the anti-HLA-G soluble scFv (FIG. 10; FIG. 10A: E:T = 5:1, FIG. 10B: E:T = 3:1).

Based on the above results, it can be seen that the decreased function of immune cells due to HLA-G expressed by the target cell line was partially restored when the anti-HLA-G soluble scFv was expressed and secreted.

### Example 7: Production of Jurkat cell line expressing anti-HLA-G soluble scFv

### 7-1: Preparation of anti-HLA-G soluble scFv gene-expressing lentivirus

Using a construct expressing the anti-HLA-G soluble scFv alone, which was produced for immune cells using endogenous antigen recognition receptors without exogenous antigen recognition receptors such as CAR, a lentivirus for delivery of these genes was produced using a plasmid DNA transfection method. A TransIT-293 transfection reagent (Mirus Bio LLC, USA) was used, and this process was performed according to the manufacturer's protocol. On the day before the experiment, Lenti-X^{™} 293T cells (Clontech, USA) were seeded at a density of 5 × 10⁶ cells in a 100 mm dish. The next day, the cells were transfected with a pLV-EF1A-soluble scFv lentiviral vector, a gag-pol expression vector, and a VSV-G envelope expression vector. The soluble scFv lentiviral vector was as follows: soluble scFv 2 or soluble scFv 3. After transfection, the cells were cultured for approximately 72 hours, and all the cell supernatants were harvested after the culture was completed. The supernatants were filtered through a 0.45 µm filter (Millipore, USA) to remove cell debris. The lentivirus was concentrated approximately 100-fold using the Lenti-X^{™} concentrator (Clontech, USA) according to the manufacturer's protocol, and stored at -80°C in a freezer until use.

### 7-2: Production and culture of anti-HLA-G soluble scFv-expressing Jurkat cell line

A Jurkat-ILT2 cell line was seeded in a 6-well plate at a density of 1 × 10⁶ cells per well along with a lentivirus having a multiplicity of infection (MOI) of approximately 5, and the culture medium was adjusted to 2 mL. Polybrene was further added to the medium at a concentration of 8 ug/mL. After static culture for 24 hours, the medium was replaced with a fresh medium. The transduced Jurkat-ILT2 cell line was maintained by subculturing at 2 × 10⁵ cells per mL at intervals of 2 to 3 days.

### 7-3: Confirmation of secretion of soluble scFv in anti-HLA-G soluble scFv-expressing Jurkat cell line

Since the soluble scFv has to be secreted out of cells to act, it was confirmed whether the soluble scFv was normally expressed and secreted out of the cells. 8 × 10⁵ cells were seeded in a 24-well plate. The next day, all the media was harvested and the protein in the media was obtained using a Minute^{™} High-Efficiency Protein Precipitation kit (Invent Biotechnologies, USA). The protein obtained through Western blotting was detected using a goat anti-human IgG antibody, Fc, HRP conjugate (Sigma-Aldrich, USA).

As a result, it was confirmed that the soluble scFv was well secreted and stably present in the Jurkat cell line expressing the soluble scFv (FIG. 11).

## Claims

1. An immune cell expressing and secreting:
an antigen recognition receptor; and
an antibody or an antigen binding fragment thereof that specifically binds to a target selected from the group consisting of human leukocyte antigen G (HLA-G) and a receptor thereof.

2. An immune cell expressing and secreting an antibody or an antigen binding fragment thereof that specifically binds to a target selected from the group consisting of human leukocyte antigen G (HLA-G) and a receptor thereof.

3. The immune cell of claim 1 or 2, wherein the antibody or antigen binding fragment thereof that specifically binds to the human leukocyte antigen G comprises a sequence selected from the following:
(a) a heavy-chain variable region comprising heavy-chain complementarity determining region 1 (CDR1) having an amino acid sequence of SEQ ID NO: 21, heavy-chain CDR2 having an amino acid sequence of SEQ ID NO: 22, and heavy-chain CDR3 having an amino acid sequence of SEQ ID NO: 23; and
a light-chain variable region comprising light-chain CDR1 having an amino acid sequence of SEQ ID NO: 24, light-chain CDR2 having an amino acid sequence of SEQ ID NO: 25, and light-chain CDR3 having an amino acid sequence of SEQ ID NO: 26;
(b) a heavy-chain variable region comprising heavy-chain CDR1 having an amino acid sequence of SEQ ID NO: 27, heavy-chain CDR2 having an amino acid sequence of SEQ ID NO: 28, and heavy-chain CDR3 having an amino acid sequence of SEQ ID NO: 29; and
a light-chain variable region comprising light-chain CDR1 having an amino acid sequence of SEQ ID NO: 30, light-chain CDR2 having an amino acid sequence of SEQ ID NO: 31, and light-chain CDR3 having an amino acid sequence of SEQ ID NO: 32; or
(c) a heavy-chain variable region comprising heavy-chain CDR1 having an amino acid sequence of SEQ ID NO: 33, heavy-chain CDR2 having an amino acid sequence of SEQ ID NO: 34, and heavy-chain CDR3 having an amino acid sequence of SEQ ID NO: 35; and
a light-chain variable region comprising light-chain CDR1 having an amino acid sequence of SEQ ID NO: 36, light-chain CDR2 having an amino acid sequence of SEQ ID NO: 37, and light-chain CDR3 having an amino acid sequence of SEQ ID NO: 38.

4. The immune cell of claim 1 or 2, wherein the antibody or antigen binding fragment thereof that specifically binds to the human leukocyte antigen G is single-chain Fv (scFv).

5. The immune cell of claim 4, wherein the single-chain Fv is secreted from immune cells.

6. The immune cell of claim 4, wherein the single-chain Fv enhances the immune response of the immune cells and tumor-infiltrating lymphocytes.

7. The immune cell of claim 1 or 2, wherein the immune cell is selected from the group consisting of natural killer cells, T lymphocytes, B lymphocytes, macrophages, dendritic cells, natural killer dendritic cells, mast cells, and precursor cells thereof.

8. The immune cell of claim 1, wherein the antigen recognition receptor is an exogenous or endogenous antigen recognition receptor.

9. The immune cell of claim 1, wherein the antigen recognition receptor is a chimeric antigen receptor (CAR) or a T-cell receptor (TCR).

10. The immune cell of claim 1, wherein the antigen recognition receptor recognizes an antigen selected from the group consisting of CD19, CD20, CD22, CD7, CD10, CD30, CD33, CD34, CD38, CD41, CD44, CD49f, CD56, CD74, CD123, CD133, CD138, CD171, an epidermal growth factor receptor (EGFR), a prostate-specific membrane antigen (PSMA), GD2, an EGFR variant, tyrosine-protein kinase transmembrane receptor 1 (ROR1), c-Met, human epidermal growth factor receptor-2 (HER2), a carcinoembryonic antigen (CEA), mesothelin, GM2, MUC16, MUC1, CS1(CD319), interleukin 13 receptor alpha 2 (IL-13R a2), a B-cell maturation antigen (BCMA), Lewis Y, an IgG kappa chain, folate receptor-alpha, a prostate stem cell antigen (PSCA), an epithelial cell adhesion molecule (EpCAM), New York esophageal squamous cell carcinoma 1 (NY-ES0-1), Wilms' tumor gene 1 (WT-1), MAGE family member A1 (MAGE-A1), tumor associated glycoprotein-72 (TAG-72), killer cell lectin like receptor K1 (NKG2D(KLRK1), Claudin 18.2, Claudin 3, Claudin 4, and Claudin 6.

11. An expression vector of any one of the following (a) to (c) for preparing the immune cell according to claim 1 or 2:
(a) an expression vector comprising a nucleotide sequence encoding an antigen recognition receptor and a nucleotide sequence encoding an antibody or an antigen binding fragment thereof that specifically binds to a target selected from the group consisting of human leukocyte antigen G and a receptor thereof;
(b) an expression vector comprising a nucleotide sequence encoding an antibody or an antigen binding fragment thereof that specifically binds to a target selected from the group consisting of human leukocyte antigen G and a receptor thereof; and
(c) two expression vectors of (c-1) and (c-2) below:
(c-1) an expression vector comprising a nucleotide sequence encoding an antigen recognition receptor; and
(c-2) an expression vector comprising a nucleotide sequence encoding an antibody or an antigen binding fragment thereof that specifically binds to a target selected from the group consisting of human leukocyte antigen G and a receptor thereof.

12. The expression vector of claim 11, wherein the expression vector of (a) is an expression vector in which a nucleotide sequence encoding an antigen recognition receptor and a nucleotide sequence encoding an antibody or an antigen binding fragment thereof that specifically binds to a target selected from the group consisting of human leukocyte antigen G and a receptor thereof are linked via a self-cleaving peptide-coding nucleotide sequence.

13. The expression vector of claim 11 or 12, wherein the nucleotide sequence encoding the antibody or antigen binding fragment thereof that specifically binds to the human leukocyte antigen G is linked to a nucleotide sequence encoding a signal sequence, a nucleotide sequence encoding an anti-HLA-G binding scFv, and a nucleotide sequence encoding an Fc region.

14. The expression vector of claim 11, wherein the nucleotide sequence encoding the antibody or antigen binding fragment thereof that specifically binds to the human leukocyte antigen G comprises:
(a) a heavy-chain variable region-coding nucleotide sequence including a nucleotide sequence encoding heavy-chain CDR1 including a nucleotide sequence encoding an amino acid sequence of SEQ ID NO: 21, 27 or 33; a nucleotide sequence encoding heavy-chain CDR2 including a nucleotide sequence encoding an amino acid sequence of SEQ ID NO: 22, 28 or 34; and a nucleotide sequence encoding heavy-chain CDR3 including a nucleotide sequence encoding an amino acid sequence of SEQ ID NO: 23, 29 or 35; and
(b) a light-chain variable region-coding nucleotide sequence including a nucleotide sequence encoding light-chain CDR1 including a nucleotide sequence encoding an amino acid sequence of SEQ ID NO: 24, 30 or 36; a nucleotide sequence encoding light-chain CDR2 including a nucleotide sequence encoding an amino acid sequence of SEQ ID NO: 25, 31 or 37; and a nucleotide sequence encoding light-chain CDR3 including a nucleotide sequence encoding an amino acid sequence of SEQ ID NO: 26, 32 or 38.

15. The expression vector of claim 14, wherein the nucleotide sequence encoding the antibody or antigen binding fragment thereof that specifically binds to the human leukocyte antigen G comprises:
(a) a nucleotide sequence encoding heavy-chain CDR1 including a nucleotide sequence encoding the amino acid sequence of SEQ ID NO: 21, a nucleotide sequence encoding heavy-chain CDR2 including a nucleotide sequence encoding the amino acid sequence of SEQ ID NO: 22, and a nucleotide sequence encoding heavy-chain CDR3 including a nucleotide sequence encoding the amino acid sequence of SEQ ID NO: 23; and
a nucleotide sequence encoding light-chain CDR1 including a nucleotide sequence encoding the amino acid sequence of SEQ ID NO: 24, a nucleotide sequence encoding light-chain CDR2 including a nucleotide sequence encoding the amino acid sequence of SEQ ID NO: 25, and a nucleotide sequence encoding light-chain CDR3 including a nucleotide sequence encoding the amino acid sequence of SEQ ID NO: 26;
(b) a nucleotide sequence encoding heavy-chain CDR1 including a nucleotide sequence encoding the amino acid sequence of SEQ ID NO: 27, a nucleotide sequence encoding heavy-chain CDR2 including a nucleotide sequence encoding the amino acid sequence of SEQ ID NO: 28, and a nucleotide sequence encoding heavy-chain CDR3 including a nucleotide sequence encoding the amino acid sequence of SEQ ID NO: 29; and
a nucleotide sequence encoding light-chain CDR1 including a nucleotide sequence encoding the amino acid sequence of SEQ ID NO: 30, a nucleotide sequence encoding light-chain CDR2 including a nucleotide sequence encoding the amino acid sequence of SEQ ID NO: 31, and a nucleotide sequence encoding light-chain CDR3 including a nucleotide sequence encoding the amino acid sequence of SEQ ID NO: 32; or
(c) a nucleotide sequence encoding heavy-chain CDR1 including a nucleotide sequence encoding the amino acid sequence of SEQ ID NO: 33, a nucleotide sequence encoding heavy-chain CDR2 including a nucleotide sequence encoding the amino acid sequence of SEQ ID NO: 34, and a nucleotide sequence encoding heavy-chain CDR3 including a nucleotide sequence encoding the amino acid sequence of SEQ ID NO: 35; and
a nucleotide sequence encoding light-chain CDR1 including a nucleotide sequence encoding the amino acid sequence of SEQ ID NO: 36, a nucleotide sequence encoding light-chain CDR2 including a nucleotide sequence encoding the amino acid sequence of SEQ ID NO: 37, and a nucleotide sequence encoding light-chain CDR3 including a nucleotide sequence encoding the amino acid sequence of SEQ ID NO: 38.

16. A pharmaceutical composition comprising the immune cell according to any one of claims 1 to 10.

17. The pharmaceutical composition of claim 16, wherein the pharmaceutical composition is used to prevent or treat cancer

18. The pharmaceutical composition of claim 17, wherein the cancer is selected from the group consisting of pancreatic cancer, breast cancer, ovarian cancer, glioma, cervical cancer, endometrial cancer, esophageal cancer, gastric cancer, liver cancer, lung cancer, colorectal cancer, nasopharyngeal cancer, oral cancer, thyroid cancer, prostate cancer, kidney cancer, gallbladder cancer, bile duct cancer, and melanoma.

19. The pharmaceutical composition of any one of claims 16 to 18, further comprising a pharmaceutically acceptable carrier.

20. A use of the immune cell according to any one of claims 1 to 10 for the preparation of a pharmaceutical composition for preventing or treating cancer.

21. A use of the immune cell according to any one of claims 1 to 10 for the prevention or treatment of cancer.

22. A method of treating cancer comprising:
administering the immune cell according to any one of claims 1 to 10 or the pharmaceutical composition according to any one of claims 16 to 18 to a subject in need thereof.
